(19) Europäisches Patentamt
European Patent Office
Office européen des brevets

(11) **EP 3 792 931 A1**

(12) **EUROPEAN PATENT APPLICATION**

(43) Date of publication:
**17.03.2021 Bulletin 2021/11**

(51) Int Cl.:
**G16H 50/20** (2018.01)  **G16H 50/70** (2018.01)
**G16H 40/20** (2018.01)

(21) Application number: **20193762.0**

(22) Date of filing: **01.09.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2019 JP 2019164352**

(71) Applicant: **Hitachi, Ltd.**
**Tokyo 100-8280 (JP)**

(72) Inventors:
• **Shibahara, Takuma**
  **Tokyo, 100-8280 (JP)**
• **Yamashita, Yasuho**
  **Tokyo, 100-8280 (JP)**
• **Nakamoto, Yoichi**
  **Tokyo, 100-8280 (JP)**

(74) Representative: **Manitz Finsterwald**
**Patent- und Rechtsanwaltspartnerschaft mbB**
**Martin-Greif-Strasse 1**
**80336 München (DE)**

(54) **DATA PROCESSING APPARATUS, DATA PROCESSING METHOD, AND DATA PROCESSING PROGRAM**

(57)    Analysis of data groups according to a combination of a plurality of elements is facilitated.

A data processing apparatus includes : a storage section storing an object-to-be-analyzed data group having factors and an objective variable per object to be analyzed; a first modulation section modulating a first factor and outputting a first modulation result per object to be analyzed; a second modulation section modulating a second factor and outputting a second modulation result per object to be analyzed; and a generation section that assigns, per object to be analyzed, a coordinate point representing the first modulation result from the first modulation section and the second modulation result from the second modulation section to a coordinate space specified by a first axis corresponding to the first factor and a second axis corresponding to the second factor, and that generates first image data obtained by assigning information associated with the objective variable of the object to be analyzed corresponding to the coordinate point to the coordinate point.

EP 3 792 931 A1

**Description**

Claim of Priority

The present application claims priority from Japanese patent application JP 2019-164352 filed on September 10, 2019, the content of which is hereby incorporated by reference into this application.

BACKGROUND OF THE INVENTION

1. Field of the Invention

The present invention relates to a data processing apparatus, a data processing method, and a data processing program for processing data.

2. Description of the Related Art

Classifying patients each contracting a disease using biological information characteristic of each patient and the disease of the patient (such as blood and gene information) so that individual medical treatment can be applied to each patient is referred to as "patient stratification" in medical terms. The patient stratification enables a medical doctor to quickly and accurately determine whether to administer a medicine to an individual patient. The patient stratification can, therefore, contribute to prompt recovery of an individual patient, lead to a reduction in medical care cost growing at an accelerated pace, and conduce to benefits of both individuals and an entire society.

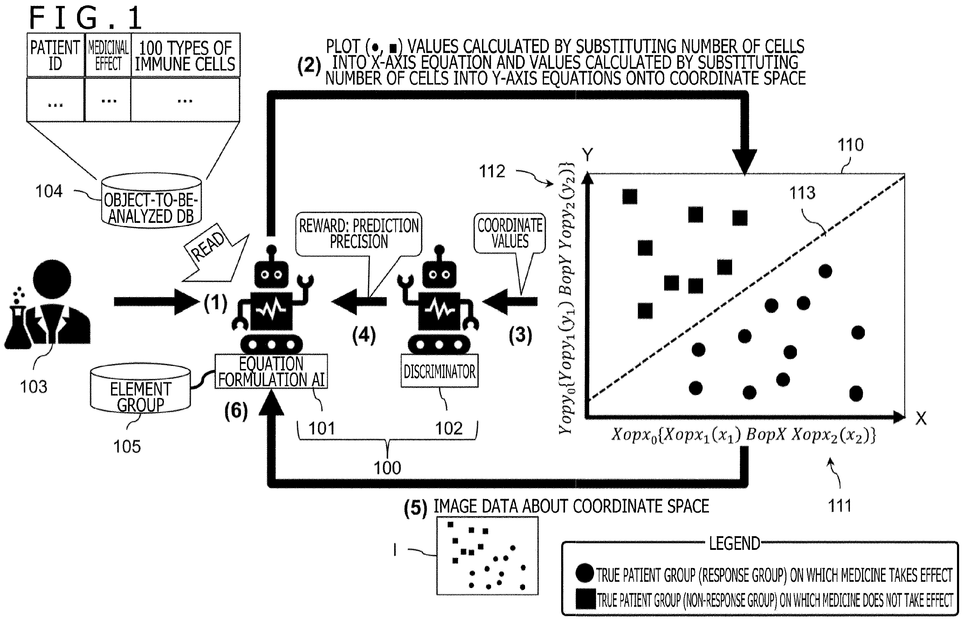

Subrahmanyam, Priyanka B., et al. "Distinct predictive biomarker candidates for response to anti-CTLA-4 and anti-PD-1 immunotherapy in melanoma patients." Journal for immunotherapy of cancer 6.1 (2018): 18., hereinafter, referred to as Non-Patent Document 1, provides a technique for stratifying skin cancer patients (melanoma patients) on the basis of characteristics of immune cells. At that time, a distribution of 40 types of immune cells depicted in Table 3 is visualized as images by a viSNE method (Figs. 1b and 1c). By visually comparing the images for a patient group (responder group) on which the medicine takes effect and a patient group (non-responder group) on which the medicine does not take effect, stratification factors are identified.

Because of complicated visual confirmation work, the technique of Non-Patent Document 1 is possibly incapable of identifying factors. Furthermore, in a case of a medicine for which patients are stratified into the responders and non-responders according to a combination of a plurality of factors, it is quite difficult to visually locate the combination from the visualized images depicted in Fig. 1c of Non-Patent Document 1.

An object of the present invention is to facilitate analyzing data groups according to a combination of a plurality of elements.

SUMMARY OF THE INVENTION

A data processing apparatus according to one aspect of the invention disclosed in the present application includes: a storage section that stores an object-to-be-analyzed data group having factors and an objective variable per object to be analyzed; a first modulation section that modulates a first factor and outputs a first modulation result per object to be analyzed; a second modulation section that modulates a second factor and outputs a second modulation result per object to be analyzed; and a generation section that assigns a coordinate point representing the first modulation result from the first modulation section and the second modulation result from the second modulation section to a coordinate space per object to be analyzed, the coordinate space being specified by a first axis corresponding to the first factor and a second axis corresponding to the second factor, and that generates first image data obtained by assigning information associated with the objective variable of the object to be analyzed corresponding to the coordinate point to the coordinate point.

According to a representative embodiment of the present invention, it is possible to facilitate analyzing data groups according to a combination of a plurality of elements. Objects, configurations, and advantages other than those described above will be readily apparent from the description of embodiments given below.

BRIEF DESCRIPTION OF THE DRAWINGS

FIG. 1 is an explanatory diagram depicting an example of analysis of a data group according to a first embodiment;
FIG. 2 is a block diagram depicting an example of a hardware configuration of a data processing apparatus;

FIG. 3 is an explanatory diagram depicting an example of an object-to-be-analyzed DB;

FIG. 4 is an explanatory diagram depicting an example of a pattern table;

FIG. 5 is a block diagram depicting an example of a circuit configuration of an image processing circuit;

FIG. 6 is a block diagram depicting an example of a configuration of a controller depicted in FIG. 5;

FIG. 7 is an explanatory diagram depicting an example of a control signal;

FIG. 8 is an explanatory diagram depicting an example of an input/output screen displayed on an output device of the data processing apparatus;

FIG. 9 is a flowchart depicting an example of detailed processing procedures of image data generation processing performed by an X-axis modulation unit, a Y-axis modulation unit, and an image generator;

FIG. 10 is a flowchart depicting an example of analysis support processing procedures;

FIG. 11 is an explanatory diagram depicting an example of a one-dimensional array;

FIG. 12 is an explanatory diagram depicting an example of an object-to-be-analyzed DB according to a second embodiment; and

FIG. 13 is an explanatory diagram depicting an example of an input/output screen displayed on an output device of a data processing apparatus according to the second embodiment.

DESCRIPTION OF THE PREFERRED EMBODIMENTS

[First embodiment]

An example of a data processing apparatus, a data analysis method, and a data analysis program according to a first embodiment will be described hereinafter with reference to the accompanying drawings. Furthermore, in the first embodiment, an object-to-be-analyzed data group is a set of object-to-be-analyzed datasets each of which is a combination of object-to-be-analyzed data indicating the number of cells of 100 types of immune cells (factor group) having a surface antigen of a medicine-administered patient and ground truth data indicating a medicinal effect of medicine administration for, for example, each of 50 patients. It is noted that the number of patients and the number of types of immune cells are given as an example.

<Example of analysis>

FIG. 1 is an explanatory diagram depicting an example of analysis of a data group according to the first embodiment. A data processing apparatus 100 has an equation formulation artificial intelligence (AI) 101 and a discriminator 102. The equation formulation AI 101 is, for example, a reinforcement learning convolutional neural network (CNN) that formulates equations 111 and 112. The discriminator 102 is an AI to which coordinate values on a coordinate space 110 specified by an X-axis and a Y-axis are input and which outputs a prediction precision as a reward to the equation formulation AI 101. A user 103 of the data processing apparatus 100 may be, for example, a medical doctor, a scholar, or a researcher, or may be a business operator providing an analysis service by the data processing apparatus 100.

(1) The user 103 selects an obj ect-to-be-analyzed data group from an obj ect-to-be-analyzed DB 104 that stores a data group for each patient and causes the equation formulation AI 101 to read the selected obj ect-to-be-analyzed data group to. The obj ect-to-be-analyzed data group is a combination of the number of cells of 100 types of immune cells and the medicinal effect per patient as described above.

(2) The equation formulation AI 101 selects two or more factors from an element group 105 and modulation methods for modulating the factors. The equation formulation AI 101 selects, for example, $\{x1, x2\}$ as X-axis factors and $\{y1, y2\}$ as Y-axis factors. Furthermore, the modulation methods are each an operator having a factor or factors as an operand or operands.

The equation formulation AI 101 formulates an X-axis equation 111 and a Y-axis equation 112 by a combination of the selected factors $\{x1, x2\}$ and $\{y1, y2\}$ and the selected modulation methods. Furthermore, the equation formulation AI 101 substitutes the number of cells identified by the patient's factors $\{x1, x2\}$ into the X-axis equation 111 to calculate an X coordinate value, substitutes the number of cells that is feature values of the patient's factors $\{y1, y2\}$ into the Y-axis equation 112 to calculate a Y coordinate value, and plots the X coordinate value and the Y coordinate value onto the coordinate space 110. The equation formulation AI 101 executes calculation of the X coordinate value and the Y coordinate value per patient.

Patients' coordinate values are plotted onto the coordinate space 110. Each black circle ● indicates coordinate values identifying a patient (response) on whom an administered medicine takes effect, while each black square ■ indicates coordinate values identifying a patient (non-response) on whom an administered medicine does not take effect. The coordinate values plotted onto the coordinate space 110 will be referred to as "patient data."

(3) The data processing apparatus 100 inputs the coordinate values as the patient data to the discriminator 102.

(4) The discriminator 102 calculates a prediction precision of a discrimination demarcation line 113 for classifying the patient data into patient data about the response and patient data about the non-response. The discriminator 102 then outputs the calculated prediction precision to the equation formulation AI 101 as a reward for reinforcement learning.

(5) Furthermore, separately from (3), the data processing apparatus 100 inputs image data I that is the coordinate space 110 onto which the patient data is plotted to the equation formulation AI 101.

(6) The equation formulation AI 101 executes convolution computation by reinforcement learning CNN on the image data I about the coordinate space 110 using the reward input in (4), and reselects factors and modulation methods configuring the equations 111 and 112 as an action to be taken next. Subsequently, the data processing apparatus 100 repeatedly executes (2) to (6).

In this way, the image data I for classifying the patient data into the patient data about the response and the patient data about the non-response with high precision is generated by causing the equation formulation AI 101 to solve the equations 111 and 112 while referring to the image data I. The user 103 can thereby easily set the high precision discrimination demarcation line 113 for classifying the patient data into the patient data about the response and the patient data about the non-response using the finally obtained image data I.

<Example of hardware configuration of data processing apparatus 100>

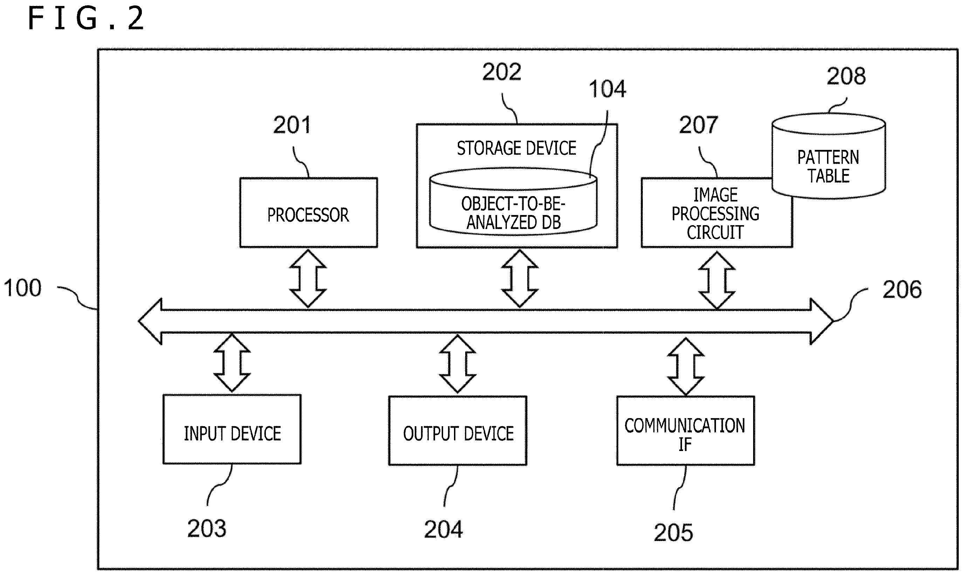

FIG. 2 is a block diagram depicting an example of a hardware configuration of the data processing apparatus 100. The data processing apparatus 100 has a processor 201, a storage device 202, an input device 203, an output device 204, a communication interface (communication IF) 205, and an image processing circuit 207. The processor 201, the storage device 202, the input device 203, the output device 204, the communication IF 205, and the image processing circuit 207 are connected by a bus 206.

The processor 201 controls the data processing apparatus 100. The storage device 202 serves as a work area for the processor 201. Furthermore, the storage device 202 is a non-transitory or transitory recording medium storing various programs and data and the object-to-be-analyzed DB. Examples of the storage device 202 include a read only memory (ROM), a random access memory (RAM), a hard disk drive (HDD), and a flash memory. The input device 203 inputs data to the data processing apparatus 100. Examples of the input device 203 include a keyboard, a mouse, a touch panel, a numeric keypad, and a scanner. The output device 204 outputs data. Examples of the output device 204 include a display and a printer. The communication IF 205 connects the data processing apparatus 100 to a network to transmit and receive data.

The image processing circuit 207 has a circuit configuration for executing stratification image processing. The image processing circuit 207 executes a series of processing (1) to (6) depicted in FIG. 1 while referring to a pattern table 208. The pattern table 208 is stored, for example, in a memory area, not depicted, within the image processing circuit 207. It is noted that while the image processing circuit 207 is realized by the circuit configuration, the image processing circuit 207 may be realized by causing the processor 201 to execute programs stored in the storage device 202.

<Object-to-be-analyzed DB 104>

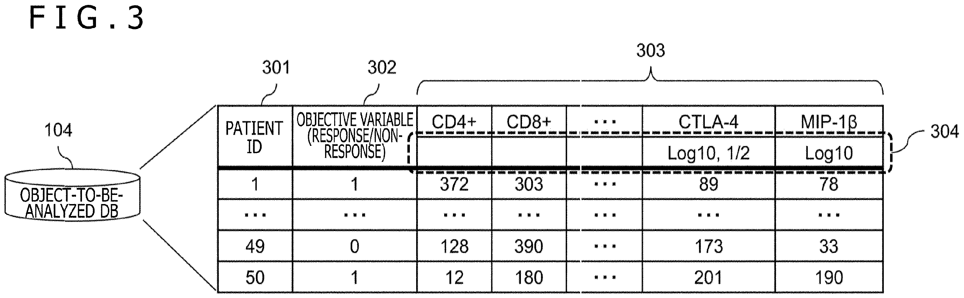

FIG. 3 is an explanatory diagram depicting an example of the object-to-be-analyzed DB 104. The object-to-be-analyzed DB 104 has a patient ID 301, an objective variable 302, and a factor group 303 as fields. A combination of values of the fields in one row is an object-to-be-analyzed dataset about one patient.

The patient ID 301 is identification information for discriminating a patient that is an example of an object to be analyzed from other patients, and a value of the patient ID 301 is expressed by, for example, 1 to 50. The objective variable 302 indicates whether a medicinal effect is present, that is, whether a medicine administration produces a response or a non-response, and a value "1" of the objective variable 302 indicates a response and a value "0" thereof indicates a non-response. The factor group 303 is a set of 100 types of factors. Each factor in the factor group 303 indicates an immune cell type. A value of the factor indicates the number of immune cells . For example, the number of cells of the factor "CD4+" of the patient ID 301 "1" is "372." In other words, each entry in the object-to-be-analyzed DB 104 indicates the medicinal effect (response or non-response) in a case of administering a medicine to the patient identified by the factor group 303.

Furthermore, a modulation method 304 is associated with each factor in the factor group 303. The modulation method 304 is an operator with the value of a factor as an operand. Types of the operator includes unary operators and multiple-operand operators. Examples of the unary operators include an identify function, a sign change, a logarithm, a square root, a sigmoid, and an arbitration function. Examples of the multiple-operand operators include four arithmetic operators.

<Pattern table 208>

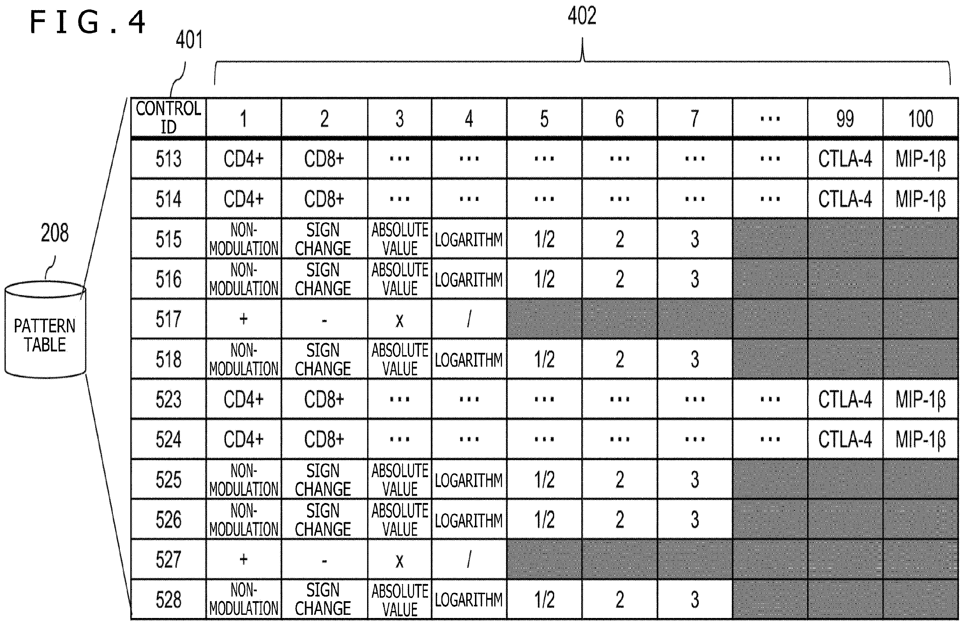

FIG. 4 is an explanatory diagram depicting an example of the pattern table 208. The pattern table 208 is a table that specifies the element group 105 used in generating a control signal for formulating the equations 111 and 112 and plotting the coordinate values onto the coordinate space 110. A content of the pattern table 208 is set in advance.

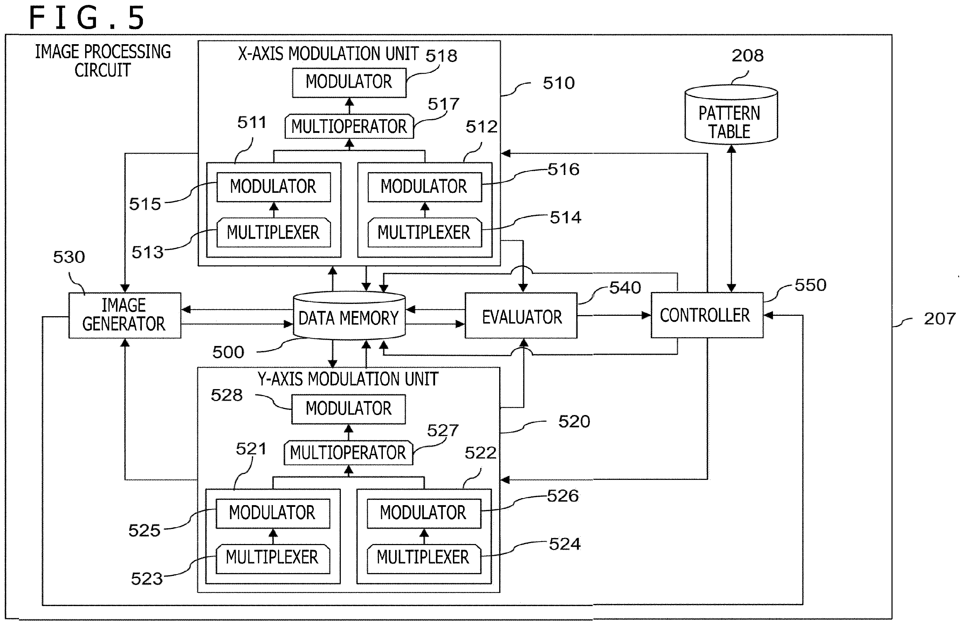

The pattern table 208 has a control ID 401 and an element number sequence 402 as fields. The control ID 401 is identification information for uniquely identifying a selection entity that selects elements (CD4+, CD8+, non-modulation, a sign change, and the like) that are values of element numbers (1 to 100) in the element number sequence 402. For the sake of convenience, it is assumed that values 513 to 518 of the control IDs 401 are reference characters assigned to modules within an X-axis modulation unit 510 of FIG. 5 to be described later. Likewise, it is assumed that values 523 to 528 of the control IDs 401 are reference characters assigned to modules within a Y-axis modulation unit 520 of FIG. 5 to be described later. The element number sequence 402 is a set of element numbers corresponding to elements selectable by each module identified by the control ID 401.

The modules having values "513," "514," "523," and "524" of the control IDs 401 each select a maximum selection number of (for example, two) factors set in advance by the data processing apparatus 100 from the factors (immune cells) that are the 100 elements . The modules indicated by the values "515" to "518," and "525" to "528" of the control IDs 401 each select any one operator from among a plurality of operators (such as the non-modulation and the sign change) that are seven or four elements. While the elements in the pattern table 208 of FIG. 4 include the types of the factors and the types of the modulation methods, the elements may include only the types of the factors or only the types of the modulation methods.

<Example of configuration of image processing circuit 207>

FIG. 5 is a block diagram depicting an example of a circuit configuration of the image processing circuit 207. The image processing circuit 207 has a data memory 500, the X-axis modulation unit 510, the Y-axis modulation unit 520, an image generator 530, an evaluator 540, a controller 550, and the pattern table 208.

All entries in the object-to-be-analyzed DB 104, that is, obj ect-to-be-analyzed datasets about patients are written to the data memory 500 from the storage device 202.

The X-axis modulation unit 510 configures part of the equation formulation AI 101 depicted in FIG. 1. The X-axis modulation unit 510 sets factors and modulation methods in the X-axis equation 111. The X-axis modulation unit 510 has X-axis data load modules 511 and 512, a multioperator 517, and a modulator 518.

The X-axis data load module 511 has a multiplexer 513 and a modulator 515. The multiplexer 513 selects a factor x1 from a control signal output from the controller 550. The multiplexer 513 may receive selection of the factor x1 selected by the user.

The modulator 515 selects a modulation method opx1 from the control signal output from the controller 550. The modulator 515 applies the modulation method opx1 to all cases related to the factor x1. A case means the number of cells of each patient for the factor x1. In a case, for example, in which the factor x1 is "CD4+," the factor x1 is a vector of x1 = (372, ..., 128, 12) indicating an array of the number of cells of 50 patients.

Examples of the modulation method opx1 to be applied include the non-modulation, the sign change, logarithmic transformation (for example, $\log_{10}$), absolute value transformation, and exponentiation. In the first embodiment, an exponent (for example, 1/2, 2, or 3) greater than 0 and not equal to 1 is incorporated for the exponentiation. It is noted that the factor x1 modulated by the modulation method opx1 is defined as "signal x1'." If the modulation method opx1 is, for example, "$\log_{10}$," the signal x1' is expressed by x1' = $\log_{10}$x1.

The X-axis data load module 512 has a multiplexer 514 and a modulator 516. Description of the X-axis data load module 512 will be omitted since the X-axis data load module 512 is identical in configuration to the X-axis data load module 511 except that the multiplexer 514 selects a factor x2 (which may be identical to x1) and that the modulator 516 selects a modulation method opx2. It is noted that the factor x2 modulated by the modulation method opx2 is defined as "signal x2'."

It is assumed in the first embodiment that the maximum selection number of X-axis factors is two. Owing to this, to facilitate understanding of the description, the two X-axis data load modules 511 and 512 are mounted in the image processing circuit 207 in FIG. 5. However, if the maximum selection number of X-axis factors is three or more, the X-axis data load modules 511 and 512 may be alternately mounted or as many data load modules as the maximum selection number of X-axis factors may be mounted. Furthermore, one X-axis data load module 511 may select a plurality of X-axis selectable factors and a plurality of operators.

The multioperator 517 selects a multiple-operand operator such as any of four arithmetic operators, a max function, and a min function from the control signal from the controller 550 as a modulation method opxa. The multioperator 517 combines the signals x1' and x2' output from the X-axis data load modules 511 and 512 by the selected modulation method opxa. The combined signal by the modulation method opxa is defined as "signal x." If the modulation method opxa is, for example, "+," the signal x is expressed by x = x1' + x2'.

The modulator 518 modulates the signal x obtained by combining by the multioperator 517 to a signal x' by a modulation method opxb. The signal x' is an X-axis coordinate value of patient data calculated by substituting the factor x1 into the X-axis equation 111. The modulator 518 stores the X-axis equation 111 and the signal x' in the data memory 500 and outputs the X-axis equation 111 and the signal x' to the image generator 530. Examples of a modulation method opxb to be applied include the non-modulation, the sign change, the logarithmic transformation (for example, $\log_{10}$), the absolute value transformation, and the exponentiation. In the first embodiment, an exponent (for example, 1/2, 2, or 3) greater than 0 and not equal to 1 is incorporated for the exponentiation. If the modulation method opxb is, for example, the exponentiation with an exponent "2," the signal x' is expressed by $x' = x^2$.

The Y-axis modulation unit 520 configures part of the equation formulation AI 101 depicted in FIG. 1. The Y-axis modulation unit 520 sets factors and modulation methods in the Y-axis equation 112. The Y-axis modulation unit 520 has Y-axis data load modules 521 and 522, a multioperator 527, and a modulator 528.

Description of the Y-axis modulation unit 520 will be omitted since the Y-axis modulation unit 520 is identical in configuration to the X-axis modulation unit 510 except that the Y-axis modulation unit 520 selects factors y1 and y2 (which may be identical to y1) as an alternative to the factors x1 and x2, selects modulation methods opy1 (modulated signal by which is signal y1'), opy2 (modulated signal by which is signal y2'), opya (modulated signal by which signal is y), and opyb (modulated signal by which is signal is y') as an alternative to the modulation methods opx1, opx2, opxa, and opxb, and generates the Y-axis equation 112 as an alternative to the X-axis equation 111.

While the X-axis modulation unit 510 and the Y-axis modulation unit 520 described above formulate the equations 111 and 112 while substituting the numbers of cells of the factors x1, x2, y1, and y2 using the control signal a(t) and obtain the coordinate values (patient data), the X-axis modulation unit 510 and the Y-axis modulation unit 520 may formulate the equations 111 and 112 first using the control signal a(t) and then obtain the coordinate values (patient data) by substituting the numbers of cells of the factors x1, x2, y1, and y2 into the formulated equations 111 and 112.

The image generator 530 configures part of the equation formulation AI 101 depicted in FIG. 1. The image generator 530 receives the signals x' and y' output from the X-axis modulation unit 510 and the Y-axis modulation unit 520. The signal x' is a set of x coordinate values (one-dimensional vector) calculated from the X-axis equation 111 per case, while the signal y' is a set of y coordinate values (one-dimensional vector) calculated from the Y-axis equation 112 per case. The image generator 530 plots the coordinate values at the same locations within the signals x' and y' onto the coordinate space 110, thereby rendering pixels that configure the image data I about the coordinate space 110 onto which the patient data is plotted.

At that time, the image generator 530 determines a color of each pixel by referring to the objective variable 302 on the data memory 500. The image generator 530 generates the image data I by, for example, rendering a response group indicated by the black circles ● of FIG. 1 in red and rendering a non-response group indicated by black squares ■ in blue. The image generator 530 stores the generated image data I in the data memory 500 and outputs the image data I to the controller 550.

The evaluator 540 has the discriminator 102 depicted in FIG. 1. The evaluator 540 acquires the signals x' and y' output from the X-axis modulation unit 510 and the Y-axis modulation unit 520 and the objective variables 302 from the data memory 500. The evaluator 540 calculates statistics r (t) in a time step t (where t is an integer equal to or greater than 1) in response to types of the objective variables 302.

Specifically, the evaluator 540 executes, for example, the discriminator 102, thereby calculating the statistics r(t) indicating the prediction precision for predicting the response or the non-response per patient. The statistics r(t) is, for example, an area under the curve (AUC) and corresponds to a reward for reinforcement learning.

A logistic regression unit, a linear regression unit, a neural network unit, a gradient boosting unit are mounted as regression calculation units as well as the discriminator 102 in the evaluator 540. The evaluator 540 stores the statistics r(t) in the data memory 500 and outputs the statistics r(t) to the controller 550.

Moreover, if the statistics r(t) is equal to or smaller than a predetermined threshold, for example, 0.5, the evaluator 540 sets a stop signal K(t) to 1, that is, K(t) = 1 and otherwise sets K(t) to zero, that is, K(t) = 0. The stop signal K(t) is a signal for determining whether to continue to generate the image data I. In a case of K(t) = 1, the evaluator 540 stops to generate the image data I; and in a case of K(t) = 0, the evaluator 540 continues to generate the image data I.

The controller 550 configures part of the equation formulation AI 101 depicted in FIG. 1. The controller 550 is a reinforcement learning CNN. The controller 550 acquires the image data I in the time step t (hereinafter, referred to as "image data I(t)") generated by the image generator 530. The controller 550 also acquires the statistics r(t) from the evaluator 540 as a reward for the reinforcement learning.

Furthermore, the controller 550 controls the X-axis modulation unit 510 and the Y-axis modulation unit 520. Specifically, when the image data I(t) is input to the controller 550 from the image generator 530, the controller 550 generates the control signal a(t) for controlling the X-axis modulation unit 510 and the Y-axis modulation unit 520 and controls generation of image data I(t+1) in a next time step (t+1).

<Configuration of controller 550>

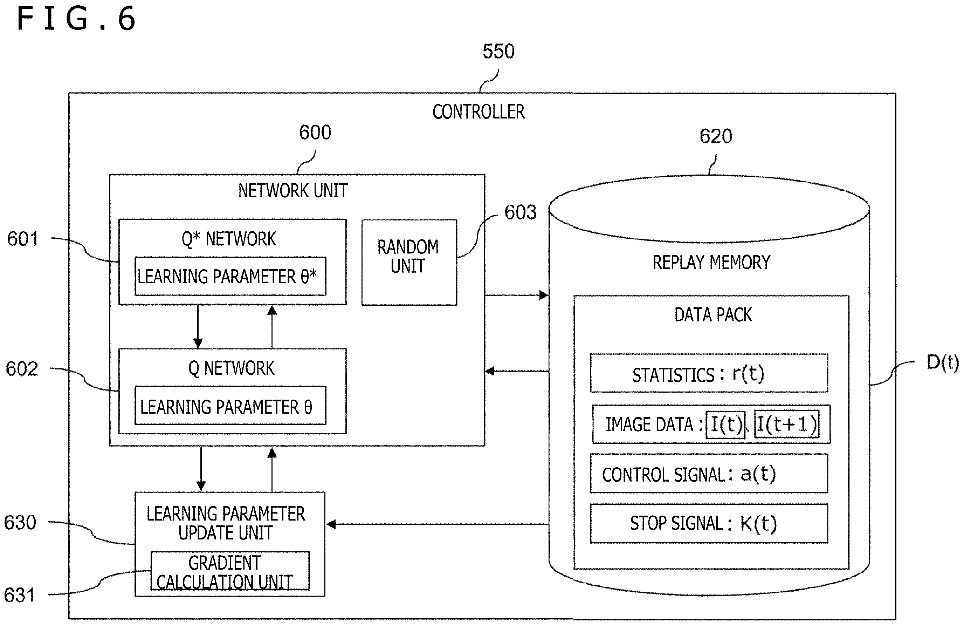

FIG. 6 is a block diagram depicting an example of a configuration of the controller 550 depicted in FIG. 5. The controller 550 has a network unit 600, a replay memory 620, and a learning parameter update unit 630. The network unit 600 has a Q* network 601, a Q network 602, and a random unit 603.

The Q* network 601 and the Q network 602 are action value functions identical in configuration for learning the control signal a(t) that is an action to maximize a value. The value in this case is an index value representing whether discrimination between a patient data group of the response and a patient data group of the non-response finally succeeds in the image data I(t) by taking an action specified by the control signal a(t) (formulating the equations 111 and 112) .

In other words, the Q* network 601 and the Q network 602 each select a maximum value of values in the element group within the pattern table 208 when taking a certain action (control signal a(t)) in a certain state (image data I(t)). In other words, the action (control signal a(t)) that enables transition into a higher value state (image data I(t+1)) has a value generally equal to a value of a next action (control signal a(t+1)).

Specifically, the Q* network 601 is a deep reinforcement learning deep Q-network (DQN) to which the image data I(t) is input and which outputs a one-dimensional array indicating values of elements (factors and modulation methods) in the control signal a(t) on the basis of a learning parameter $\theta^*$.

The Q network 602 is a deep reinforcement learning DQN identical in configuration to the Q* network 601, and obtains values of elements (combination of factors and modulation methods) serving as a generation source for the image data I(t) using a learning parameter $\theta$. The Q* network 601 selects an action highest in the value of the image data I(t) obtained by the Q network 602, that is, an element in the pattern table 208.

The random unit 603 outputs a random number value that serves as a threshold for determining whether to continue to generate the image data I(t) and that is equal to or greater than 0 and equal to or smaller than 1. The learning parameter update unit 630 has a gradient calculation unit 631. The learning parameter update unit 630 calculates a gradient g taking into account the statistic r(t) as a reward using the gradient calculation unit 631, and adds the gradient g to the learning parameter $\theta$, thereby updating the learning parameter $\theta$.

The replay memory 620 stores a data pack D(t). The data pack D(t) contains the statistic r(t), the image data I(t) and I(t+1)), the control signal a(t), and the stop signal K(t) in the time step t. In the data pack D(t), a state of a time step t+1 generated in the case of taking the action (control signal a(t)) in the state (image data I(t)) in the time step t is the image data I(t+1), and the reward obtained in the case of taking the action (control signal a(t)) is the statistics r(t); thus, the data pack D(t) identifies whether to continue to generate the image data I (t) and I(t+1) in the next time step t = t + 1 (stop signal K(t)).

An example of a configuration of the Q* network 601 will be specifically described. The Q* network 601 will be described while taking a case of inputting color image data I(t) of 84×84 pixels to the Q* network 601 by way of example. The example of the configuration of the Q* network 601 will be described. A first layer is a convolutional network (kernel: 8×8 pixels, stride: 4, and activation function: ReLU) . A second layer is a convolutional network (kernel: 4×4 pixels, stride: 2, and activation function: ReLU).

A third layer is a fully connected network (number of neurons: 256 and activation function: ReLU). Furthermore, an output layer is a fully connected network and outputs a one-dimensional array z(t) corresponding to an element sequence in the pattern table 208 as an output signal. Items of the one-dimensional array z(t) as the output signal will be described.

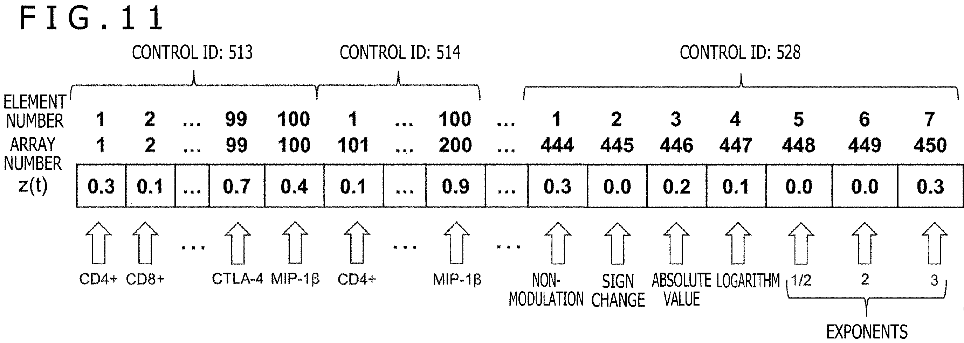

The one-dimensional array z(t) has values each corresponding to each element by one-to-one in the pattern table 208 in order of the multiplexer 513: 100 elements, the multiplexer 514: 100 elements, the modulator 515: seven elements, the modulator 516: seven elements, the multioperator 517: four elements, the modulator 518: seven elements, the multiplexer 523: 100 elements, the multiplexer 524: 100 elements, the modulator 525: seven elements, the modulator 526: seven elements, the multioperator 527: four elements, and the modulator 528: seven elements (450 elements in total) . In other words, the one-dimensional array z(t) is an array having the values corresponding to the 450 elements (refer to FIG. 11).

<Control signal a(t)>

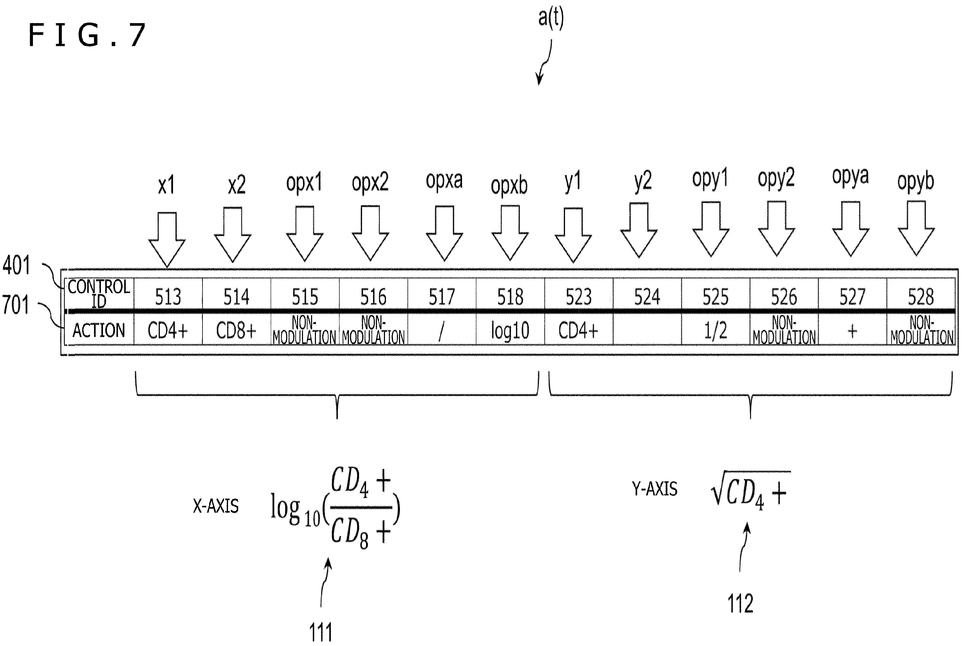

FIG. 7 is an explanatory diagram depicting an example of the control signal a(t). The control signal a(t) has a control ID 401 and an action 701 as fields. Each action 701 indicates selection of a factor or a modulation method by the X-axis modulation unit 510 or the Y-axis modulation unit 520. Each of the modules 513 to 518 and 523 to 528 designated by the control ID 401 selects a factor or a modulation method in accordance with the action 701. For example, the multiplexer 513 that has the control ID 401 "513" selects the immune cell "CD4+" as the factor x1. Therefore, the multiplexer 513 reads the number of cells (372, ..., 128, 12) in a CD4+ column from the object-to-be-analyzed DB 104 within the data memory 500.

Furthermore, the modulator 515 having the control ID 401 "515" selects "non-modulation" as the modulation method (operator opx1). Therefore, the modulator 518 modulates the number of cells in the CD4+ column (372, ..., 128, 12) read as the factor x1 from the obj ect-to-be-analyzed DB 104 within the data memory 500 to the signal x1'.

Moreover, the multiplexer 524 having the control ID 401 "524" does not select the factor y2 since the action 701 is blank. Furthermore, the modulator 525 having the control ID 401 "525" selects "1/2" (square root, one-half power) as the modulation method opy1. Therefore, the modulator 528 transforms the numbers of cells in the CD4+ column (372, ..., 128, 12) read from the object-to-be-analyzed DB 104 within the data memory 500 as the factors y1 into square roots of the numbers of cells ($\sqrt{372}$, ..., $\sqrt{128}$, $\sqrt{12}$), and obtains the signal y1'.

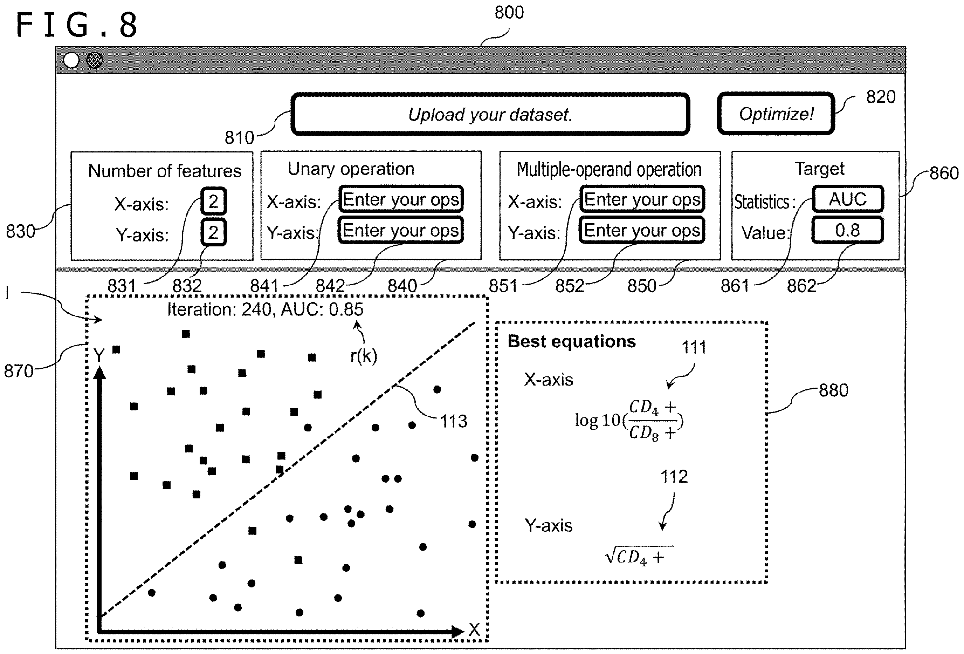

The X-axis equation 111 and the Y-axis equation 112 generated in the case of giving the control signal a(t) depicted in FIG. 7 are depicted in FIG. 7. Values of "CD4+" (372, ..., 127, 12) of the patient IDs 301 depicted in FIG. 3 are substituted into "CD4+" in each of the equations 111 and 112, and values of "CD8+" (303, ..., 390, 180) of the patient IDs 301 depicted in FIG. 3 are substituted into "CD8+" in the equation 111. It is noted that the control signal a(t) in t = 1 may be set at random from the pattern table 208 or may be set by the user 103 in FIG. 8 to be described later.

<Example of input/output screen>

FIG. 8 is an explanatory diagram depicting an example of an input/output screen displayed on the output device 204 of the data processing apparatus 100. An input/output screen 800 contains a load button 810, a start button 820, a number-of-factors input area 830, a unary operator input area 840, a multiple-operand operator input area 850, a target measure input area 860, an image display area 870, and an equation display area 880.

The load button 810 is a button for loading entries in the object-to-be-analyzed DB 104 to the data memory 500 by being depressed. The start button 820 is a button for starting stratification image generation by being depressed.

The number-of-factors input area 830 has a number-of-X-axis-factors input area 831 and a number-of-Y-axis-factors input area 832. The number of X-axis factors can be input to the number-of-X-axis-factors input area 831. In a case in which the number-of-X-axis-factors input area 831 is blank, a numeric value equal to or greater than 1 and equal to or smaller than the maximum number of factors (2 in the present embodiment) is automatically set. The number of Y-axis factors can be input to the number-of-Y-axis-factors input area 832. In a case in which the number-of-Y-axis-factors input area 832 is blank, a numeric value equal to or greater than 1 and equal to or smaller than the maximum number of factors (2 in the present embodiment) is automatically set. It is noted that the maximum number of factors can be changed on a setting screen that is not depicted.

The unary operator input area 840 includes an X-axis unary operator input area 841 and a Y-axis unary operator input area 842. A unary operator that is one of the modulation methods for the X-axis can be additionally input to the X-axis unary operator input area 841 for each of the modulators 515, 516, and 518. Likewise, a unary operator that is one of the modulation methods for the Y-axis can be additionally input to the Y-axis unary operator input area 842 for each of the modulators 525, 526, and 528.

A trigonometric function, for example, unregistered in the pattern table 208 can be additionally input to any of the X-axis unary operator input area 841 and the Y-axis unary operator input area 842 as the unary operator that can be additionally input. In a case in which the trigonometric function is not additionally input, the unary operator (the non-modulation, the sign change, the absolute value, the logarithm, or the exponent (1/2, 2, or 3)) registered in the pattern table 208 is applied.

The multiple-operand operator input area 850 includes an X-axis multiple-operand operator input area 851 and a Y-axis multiple-operand operator input area 852. A multiple-oper and operator that is one of the modulation methods for the X-axis can be additionally input to the X-axis multiple operators input area 851 for the multioperator 517. Likewise, a multiple-operand operator that is one of the modulation methods for the Y-axis can be additionally input to the Y-axis multiple-operand operator input area 852 for the multioperator 527. For example, a max function or a min function unregistered in the pattern table 208 can be additionally input as the multiple-operand operator that can be additionally input. In a case in which the max function or the min function is not additionally input, the multiple-operand operator (+, -, ×, or /) registered in the pattern table 208 is applied.

The target measure input area 860 contains a statistic input area 861 and a target value input area 862. A type of the statistics to be calculated by the learning parameter update unit 630 can be input to the statistic input area 861. Specifically, the statistics which is, for example, the AUC for determining whether the response/non-response is positive or negative can be selected. A target value (for example, "0.8" in FIG. 8) of the statistics input to the statistic input area 861 can be input to the target value input area 862.

The image data I generated by the image generator 530 is displayed in the image display area 870. For example, the image generator 530 renders the response group indicated by the black circles ● in red and renders the non-response group indicated by black squares ■ in blue. The discrimination demarcation line 113 is calculated by the discriminator 102. The X-axis equation 111 and the Y-axis equation 112 are displayed in the equation display area 880.

It is noted that the input/output screen 800 is displayed, for example, on a display that is an example of the output device 204 in the data processing apparatus 100. Alternatively, the input/output screen 800 may be displayed on a display of the other computer communicably connected to the communication IF 205 of the data processing apparatus 100 by transmitting information associated with the input/output screen 800 from the communication IF 205 to the other computer.

<Image data generation processing>

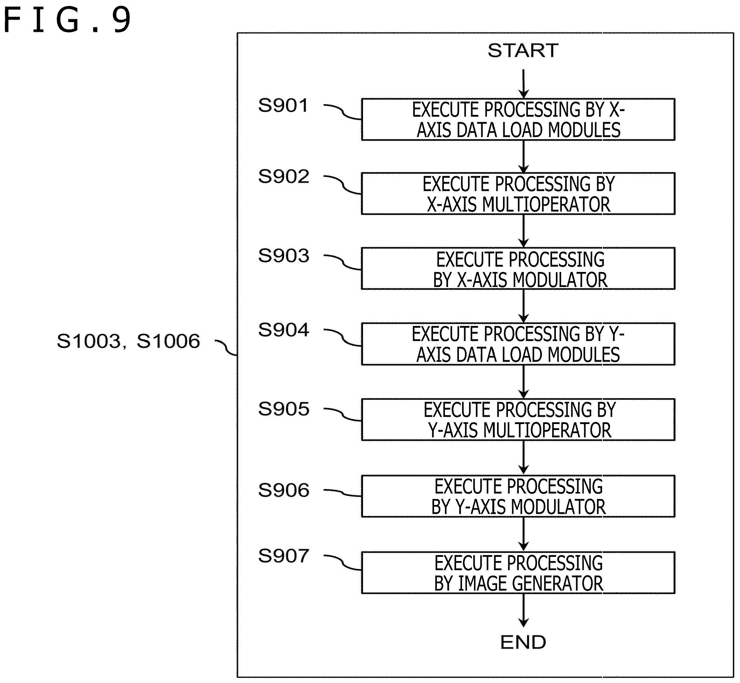

FIG. 9 is a flowchart depicting an example of detailed processing procedures of image data generation processing performed by the X-axis modulation unit 510, the Y-axis modulation unit 520, and the image generator 530. First, the X-axis data load modules 511 and 512 in the X-axis modulation unit 510 execute processing (Step S901). Specifically, the multiplexer 513 incorporated into the X-axis data load module 511, for example, selects one factor x1 from the factor group 303 stored in the data memory 500 by the control signal a(t) from the controller 550.

Next, the modulator 515 applies the modulation method designated by the control signal a (t) to all cases of the factor x1 (numbers of cells of the factor x1), and generates the signal x1'. It is noted that the modulation method 304 may be preferentially applied in a case of setting the modulation method 304 to the selected factor x1. When MIP-1$\beta$, for example, is selected as the factor x1, the factor x1 is modulated by $\log_{10}$. Furthermore, when CTLA-4 is selected as the factor x1, the factor x1 is modulated by either $\log_{10}$ or the square root (one-half power).

It is noted that the modulator 515 may preferentially apply the unary operator (for example, trigonometric function) input to the X-axis unary operator input area 841 when the unary operator is input to the X-axis unary operator input area 841. While the processing performed by the X-axis data load module 511 has been described in relation to Step S901, another X-axis data load module 512 similarly performs processing.

The multioperator 517 combines the signal x1' obtained by modulation by and output from the X-axis data load module 511 and the signal x2' obtained by modulation by and output from the X-axis data load module 512 into the signal x in accordance with the control signal a (t) (Step S902) . In a case in which the modulation method designated by the control signal a (t) is addition (+), the multioperator 517 adds up the signals x1' and x2' (x = x1' + x2').

Alternatively, when the multiple-operand operator (for example, max function) is input to the X-axis multiple-operand operator input area 851, the multioperator 517 selects a signal having a greater value out of the signals x1' and x2' as the signal x. The signals x1' and x2' are each a one-dimensional vector having modulated values corresponding to the number of patients (50 cases) . Therefore, in a case of comparing the signal x1' with the signal x2', the multioperator 517 may compare maximum values and select the signal having the greater maximum value as the signal x. In another alternative, the multioperator 517 may compare total values and select the signal having the greater total value as the signal x.

In yet another alternative, the multioperator 517 may compare values of the same patients in the signals x1' and x2' and select the signal having the larger number of greater values as the signal x. Likewise, in a case in which the multiple-operand operator is the min function and the signal x1' is compared with the signal x2', the multioperator 517 may compare minimum values and select the signal having the smaller minimum value as the signal x. In another alternative, the multioperator 517 may compare total values and select the signal having the smaller total value as the signina x. In yet another alternative, the multioperator 517 may compare values of the same patients in the signals x1' and x2' and select the signal having the larger number of smaller values as the signal x.

The modulator 518 modulates the signal x obtained by combining by the multioperator 517 in accordance with the control signal a(t), outputs the signal x' that is the X-axis coordinate value of each patient calculated by the X-axis equation 111, stores the signal x' in the data memory 500, and outputs the signal x' to the image generator 530 (Step S903). In a case in which the modulation method opxb designated by the control signal a(t) is the sign change, the modulator 518 changes a sign of the signal x.

It is noted that the modulator 518 may preferentially apply the unary operator (for example, trigonometric function) input to the X-axis unary operator input area 841 to the signal x when the unary operator is input to the X-axis unary operator input area 841.

The Y-axis data load modules 521 and 522 in the Y-axis modulation unit 520 execute processing (Step S904). The multiplexer 523 incorporated into the data load module 521 selects one factor y1 from the factor group 303 stored in the data memory 500 by the control signal a(t).

Next, the modulator 525 applies the modulation method designated by the control signal a (t) to all cases of the factor y1 (numbers of cells of the factor y1), and generates the signal y1'. It is noted that the modulation method 304 may be preferentially applied in a case of setting the modulation method 304 to the selected factor y1. When MIP-1$\beta$, for example, is selected as the factor y1, the factor y1 is modulated by $\log_{10}$. Furthermore, when CTLA-4 is selected as the factor y1, the factor y1 is modulated by either $\log_{10}$ or the square root (one-half power).

It is noted that the modulator 525 may preferentially apply the unary operator (for example, trigonometric function) input to the Y-axis unary operator input area 842 when the unary operator is input to the Y-axis unary operator input area 842. While the processing performed by the Y-axis data load module 521 has been described in relation to Step S904, another Y-axis data load module 522 similarly performs processing.

The multioperator 527 combines the signal y1' obtained by modulation by and output from the Y-axis data load module 521 and the signal y2' obtained by modulation by and output from the Y-axis data load module 522 into the signal y in accordance with the control signal a (t) (Step S905) . In a case in which the modulation method designated by the control signal a(t) is subtraction (-), the multioperator 527 subtracts the signal y2' from the signal y1' (y = y1' - y2').

Alternatively, when the multiple-operand operator (for example, max function) is input to the Y-axis multiple-operand operator input area 852, the multioperator 527 selects a signal having a greater value out of the signals y1' and y2' as the signal y. The signals y1' and y2' are each a one-dimensional vector having modulated values corresponding to the number of patients (50 cases) . Therefore, in a case of comparing the signal y1' with the signal y2', the multioperator 527 may compare maximum values and select the signal having the greater maximum value selected as the signal y.

In another alternative, the multioperator 527 may compare values of the same patients in the signals y1' and y2' and select the signal having the larger number of greater values as the signal y. Likewise, in a case in which the multiple-operand operator is the min function and the signal y1' is compared with the signal y2', the multioperator 527 may compare minimum values and select the signal having the smaller minimum value as the signal y. In another alternative, the multioperator 527 may compare values of the same patients in the signals y1' and y2' and select the signal having the larger number of smaller values as the signal y.

The modulator 528 modulates the signal y obtained by combining by the multioperator 527 to the signal y' in accordance with the control signal a(t), stores the signal y' in the data memory 500, and outputs the signal y' to the image generator 530 (Step S906). In a case in which the modulation method opyb designated by the control signal a(t) is the sign change, the modulator 528 changes a sign of the signal y.

It is noted that the modulator 528 may preferentially apply the unary operator (for example, trigonometric function) input to the Y-axis unary operator input area 842 when the unary operator is input to the Y-axis unary operator input area 842.

The image generator 530 plots the coordinate values per patient onto the coordinate space 110 on the basis of the signals x' and y' output from the X-axis modulation unit 510 and the Y-axis modulation unit 520, and generates the image data I (t) (Step S907) . At that time, the image generator 530 determines a color of each pixel by referring to the objective variable 302 on the data memory 500.

<Example of analysis processing procedures>

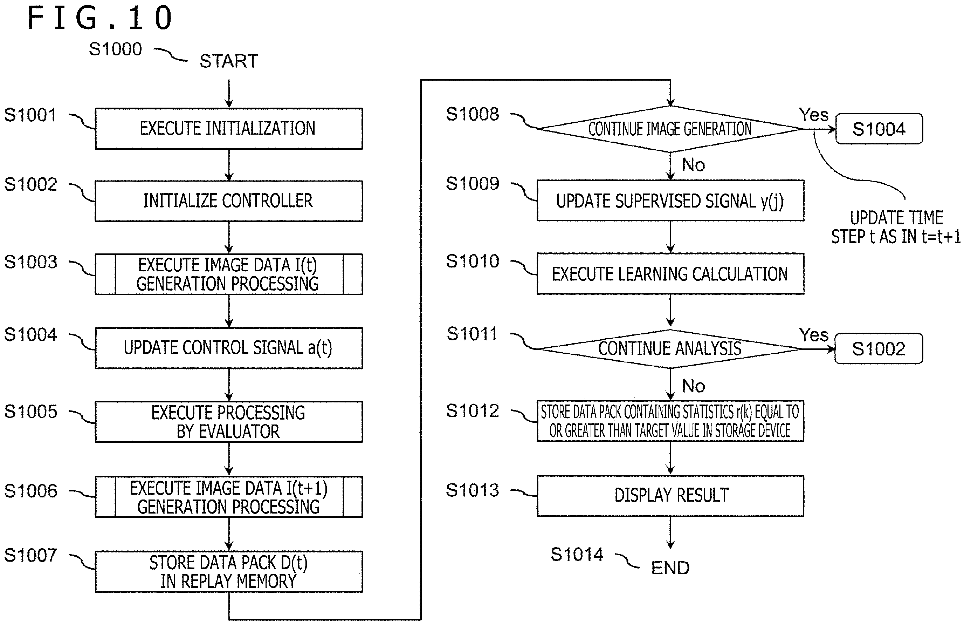

FIG. 10 is a flowchart depicting an example of analysis support processing procedures. It is assumed that entries in the obj ect-to-be-analyzed DB 104 are loaded to the data memory 500 by depressing the load button 810 on the input/output screen 800 of FIG. 8 before start of processing.

[S1001]

The data processing apparatus 100 executes initialization (Step S1001). Specifically, the data processing apparatus 100 sets a calculation step m to, for example, 1, that is, m = 1. In addition, the data processing apparatus 100 initializes the learning parameter θ* of the Q* network 601 with a random weight. Furthermore, the data processing apparatus 100 initializes the learning parameter θ of the Q network 602 with a random weight.

[S1002]

The data processing apparatus 100 initializes the controller 550 (Step S1002). Specifically, the data processing apparatus 100 sets the time step t to, for example, 1, that is, t = 1. The controller 550 sets the control signal a(t) at random using the elements in the pattern table 208.

[S1003]

Next, the data processing apparatus 100 executes the image data generation processing (hereinafter, referred to as "image data I(t) generation processing") depicted in FIG. 9 in the time step t as a subroutine (Step S1003) . In the image data I(t) generation processing (Step S1003), the image generator 530 generates the image data I(t) by giving the control signal a(t) to the X-axis modulation unit 510 and the Y-axis modulation unit 520.

[S1004]

The controller 550 updates the control signal a(t) in the time step t generated in Step S1002 (Step S1004). Specifically, the random unit 603 outputs, for example, a random number value. When the random number value output by the random unit 603 is equal to or greater than e (for example, e = 0.5), the controller 550 selects one element from the pattern table 208 at random and updates the control signal a(t) using the selected element.

The element selected at random from the pattern table 208 is, for example, "CTLA-4" of the element number 99 in the entry having the control ID 401 "513," the controller 550 changes a value "CD4+" in the action 701 indicated by the control ID 401 "513" in the control signal a(t) of FIG. 7 to "CTLA-4."

The element selected at random from the pattern table 208 is, for example, "sign change" of the element number 2 in the entry having the control ID 401 "515," the controller 550 changes a value "non-modulation" in the action 701 indicated by the control ID 401 "515" in the control signal a(t) of FIG. 7 to "sign change." It is noted that the number of elements selected at random is not limited to one but may be two or more.

On the other hand, the random number value output by the random unit 603 is smaller than e, the controller 550 inputs the image data I(t) generated in the image data I(t) generation processing (Step S1003) to the Q* network 601 in the network unit 600 and calculates the one-dimensional array z(t).

<One-dimensional array z(t)>

FIG. 11 is an explanatory diagram depicting an example of the one-dimensional array z(t). The one-dimensional array z (t) is an array of 450 numerical values corresponding to the element group of 450 elements in the pattern table 208. A magnitude of each numerical value indicates a selection value of the corresponding element. Array numbers indicate array positions of the numerical values, respectively, and correspond to arrays of all elements in the pattern table 208. For example, array numbers 1 to 100 correspond to the element numbers 1 to 100 of the control ID 401: 513. The array numbers 101 to 200 correspond to the element numbers 1 to 100 of the control ID 401: 514.

Although not depicted, array numbers 201 to 207 correspond to the element numbers 1 to 7 of the control ID 401: 515, array numbers 208 to 214 correspond to the element numbers 1 to 7 of the control ID 401: 516, array numbers 215 to 218 correspond to the element numbers 1 to 4 of the control ID 401: 517, array numbers 219 to 225 correspond to the element numbers 1 to 7 of the control ID 401: 518, array numbers 226 to 325 correspond to the element numbers 1 to 100 of the control ID 401: 523, array numbers 326 to 425 correspond to the element numbers 1 to 100 of the control ID 401: 524, array numbers 426 to 432 correspond to the element numbers 1 to 7 of the control ID 401: 525, array numbers 433 to 439 correspond to the element numbers 1 to 7 of the control ID 401: 526, and array numbers 440 to 443 correspond to the element numbers 1 to 4 of the control ID 401: 527.

In this way, the array numbers are allocated in sequence in ascending order to correspond to the elements in ascending order of the control IDs 401, and array numbers 444 to 450 correspond to the element numbers 1 to 7 of the last control ID 401: 528.

The controller 550 selects one element in the pattern table 208 corresponding to the element having the maximum value in the one-dimensional array z(t), and updates the control signal a(t). In FIG. 11, the maximum value is, for example, "0.9" of the array number 200. The array number 200 corresponds to the control ID 401: 514 and the element number 100.

In the pattern table 208, the element corresponding to the control ID 401: 514 and the element number 100 is "MIP-1β." The controller 550 changes the value "CD8+" in the action 701 indicated by the control ID 401 "514" in the control signal a(t) of FIG. 7 to "MIP-1β" corresponding to the maximum value. In this way, changing the element to the element having the maximum value makes it possible to enhance a value of the changed control signal a(t) and makes it possible for the controller 550 to take a more appropriate action, whereby the image generator 530 can generate the image data I(t) for which the arrays of the coordinate values (patient data) on the coordinate space 110 are more suited for discrimination and regression analysis.

Furthermore, in a case in which a plurality of elements having the maximum value are present, the controller 550 may select all elements or select one from among the elements at random. Moreover, the controller 550 may select not only the element or elements having the maximum value but also elements having numerical values magnitudes of which are top n (where n is an optional integer equal to or greater than 1) numerical values. In this case, the controller 550 may also select all top n elements or select one from among those elements at random.

Furthermore, the controller 550 may select the elements the magnitudes of numerical values of which are equal to or greater than a threshold. In this case, the controller 550 may also select all elements having the magnitudes of numerical values equal to or greater than the threshold or select one from among those elements at random. Moreover, the controller 550 may sequentially holds a one-dimensional array z(t-1) in a time step t-1, and select the elements each having a numerical value greater than a numerical value of the element in the one-dimensional array z(t-1) from the one-dimensional array z(t). In this case, similarly to the above, the controller 550 may select all elements each having the numerical value greater than that of the element in the one-dimensional array z(t-1) or select one from among those elements at random. In this way, the values of the elements improve as generation of the one-dimensional array z(t) is more repeated.

[S1005]

Reference is made back to FIG. 10. The evaluator 540 executes calculation of the statistics r(t) in the time step t (Step S1005). Specifically, the evaluator 540 calculates the statistics r(t) on the basis of, for example, the signals x' and y' output from the X-axis modulation unit 510 and the Y-axis modulation unit 520 and the types of the objective variables 302 loaded from the data memory 500.

More specifically, the evaluator 540 predicts the response or the non-response per patient and calculates the statistics r(t) by executing the discriminator 102. The evaluator 540 stores the statistics r(t) in the data memory 500 and outputs the statistics r(t) to the controller 550. Furthermore, if the statistics r(t) is equal to or smaller than 0.5, the evaluator 540 determines that it is impossible to generate the image data I(t) in which the response and the non-response are easy to discriminate with the element group that can be designated by the current control signal a(t), and sets the stop signal K(t) to 1, that is, K(t) = 1 (stop to generate the image data I(t)). If the statistics r(t) is not equal to and not smaller than 0.5, the evaluator 540 sets the stop signal K(t) to 0, that is, K(t) = 0 (continue to generate the image data I(t)).

[S1006]

Next, the data processing apparatus 100 executes the image data generation processing (hereinafter, referred to as "image data I(t+1) generation processing") depicted in FIG. 9 in the time step t+1 as a subroutine (Step S1006) . In the image data I(t+1) generation processing (Step S1006), the image generator 530 generates the image data I(t+1) by giving the control signal a(t) updated in Step S1004 or the control signal a(t) updated in Step S1004 in the time step t that is updated to the next time step t+1 after Step S1008: Yes, to the X-axis modulation unit 510 and the Y-axis modulation unit 520.

[S1007]

Next, the network unit 600 stores the data pack D(t) that is a set of data containing the statistics r(t), the control signal a (t), the image data I(t), the image data I(t+1), and the stop signal K (t) in the replay memory 620 (Step S1007) .

[S1008]

Furthermore, when K (t) = 0 and the time step t is smaller than a predetermined number of times T (Step S1008: Yes), the generation of the image data I(t) continues; thus, t is set to t+1, that is, t = t + 1, the time step t is updated, and the processing returns to Step S1004. On the other hand, when K(t) = 1 or the time step t is equal to or greater than the predetermined number of times T (Step S1008: No), the processing goes to Step S1009. In the first embodiment, it is assumed that T = 100.

[S1009]

The learning parameter update unit 630 loads J data packs D(1), ..., D(j), ..., and D(J) (where j = 1 to J) (hereinafter, referred to as "data pack group Ds") at random from the replay memory 620, and updates a supervised signal y(j) as represented by the following Equations (1) (Step S1009) . It is noted that an upper limit of J is assumed as 100 in the first embodiment.

[Expression 1]

$$\begin{cases} y(j) = r(j), & if\, K(j) = 1 \\ y(j) = r(j) + \gamma max\, Q(I(j+1); \theta), & otherwise \end{cases} \quad ...(1)$$

In Equations (1), $\gamma$ indicates a discount rate and assumed as $\gamma = 0.998$ in the first embodiment. Calculation processing maxQ(I(j + 1);$\theta$) in Equations (1) is processing for inputting image data I(j+1) to the Q network 602 in the network unit 600 and outputting a maximum value, that is, a maximum action value from within a one-dimensional array z(j) calculated by the Q network 602 while applying the learning parameter $\theta$. In a case, for example, in which the one-dimensional array z(t) of FIG. 11 is the one-dimensional array z(j), the value "0.9" of the array number 200 is output as the maximum action value in the calculation processing maxQ(I(j + 1);$\theta$).

[S1010]

Next, the learning parameter update unit 630 executes learning calculation (Step S1010). Specifically, the gradient calculation unit 631 updates the learning parameter θ by, for example, outputting the gradient g for the learning parameter θ using the following Equation (2) and adding the gradient g to the learning parameter θ.
[Expression 2]

$$\theta = \theta + \left( y(j) - Q(I(j); \theta) \right)^2 \quad ...(2)$$

The gradient g corresponds to a second term on a right side of Equation (2). The Q network 602 can thereby generate the control signal a(t) indicating the statistics r(t), that is, the action 701 for enhancing the prediction precision for the response or the non-response of each patient by the updated learning parameter θ taking into account the statistics r(t) that is the reward.

Furthermore, in the learning calculation (Step S1010), the learning parameter update unit 630 overwrites the updated learning parameter θ of the Q network 602 on the learning parameter θ* of the Q* network 601. In other words, the learning parameter θ* is made identical in value to the updated learning parameter θ. The Q* network 601 can thereby identify an action value, that is, the action 701 for enabling the arrangement of the patient data on the coordinate space 110 to facilitate discriminating the response and the non-response.

[S1011]

Next, when the statistics r(t) falls below the target value input to the target value input area 862 and the calculation step m is smaller than the predetermined number of times M (Step S1011: Yes), the data processing apparatus 100 returns to Step S302 and updates the calculation step m as in m = m + 1 for continuing analysis by the data processing apparatus 100. In the first embodiment, it is assumed that M = one million.

On the other hand, in a case in which the statistics r(t) is equal to or greater than the target value input to the target value input area 862 or the calculation step m reaches the predetermined number of times M (Step S1011: No), the data processing apparatus 100 goes to Step S1012.

[S1012]

Next, the data processing apparatus 100 stores a data pack D(k) in a time step k in which statistics r(k) is equal to or greater than the target value among the data pack group Ds stored in the data memory 500, in the storage device 202 (Step S1012). In a case in which the data pack D(k) in the time step k in which the statistics r (k) is equal to or greater than the target value is not present, the data processing apparatus 100 does not store the data pack D(k) in the storage device 202. Alternatively, in the case in which the data pack D(k) in the time step k in which the statistics r(k) is equal to or greater than the target value is not present, the data processing apparatus 100 may store the data pack D(k) in the time step k in which the statistics r(k) is maximum among the data pack group Ds in the storage device 202.

[S1013]

Next, the data processing apparatus 100 displays an analysis result (Step S1013). Specifically and for example, the data processing apparatus 100 loads the data pack D(k) stored in the storage device 202, causes the X-axis modulation unit 510 and the Y-axis modulation unit 520 to execute formulating the equations using a control signal a(k) in the data pack D(k), and displays the formulated equations 111 and 112 in the equation display area 880.

Furthermore, the data processing apparatus 100 displays image data I(k) and the statistics r(k) in the data pack D(k) in the image display area 870. Moreover, the data processing apparatus 100 displays the discrimination demarcation line 113 calculated by the discriminator 102 in the image display area 870. It is noted that the data processing apparatus 100 may display an analysis result indicating a failure in analysis in a case in which the data pack D(k) is not stored in the storage device 202. A series of processing is thereby ended (Step S1014).

In this way, the first embodiment can automatically discriminate the data groups according to a combination of a plurality of factors at high speed.

[Second embodiment]

A second embodiment is an example in which the objective variable 302 of the first embodiment is a quantitative variable. To mainly describe differences from the first embodiment, the same configurations as those in the first embodiment are denoted by the same reference characters and description thereof will be omitted.

<Object-to-be-analyzed DB 1200>

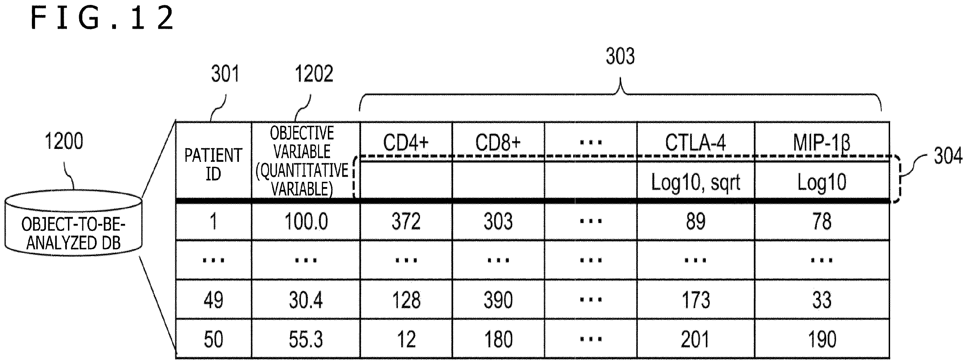

FIG. 12 is an explanatory diagram depicting an example of an object-to-be-analyzed DB 1200 according to the second embodiment. The object-to-be-analyzed DB 1200 has an objective variable 1202 that is a quantitative variable as a field as an alternative to the objective variable 302. A magnitude (major axis) in mm of a tumor of each patient is stored in each objective variable 1202 as a value.

<Example of input/output screen>

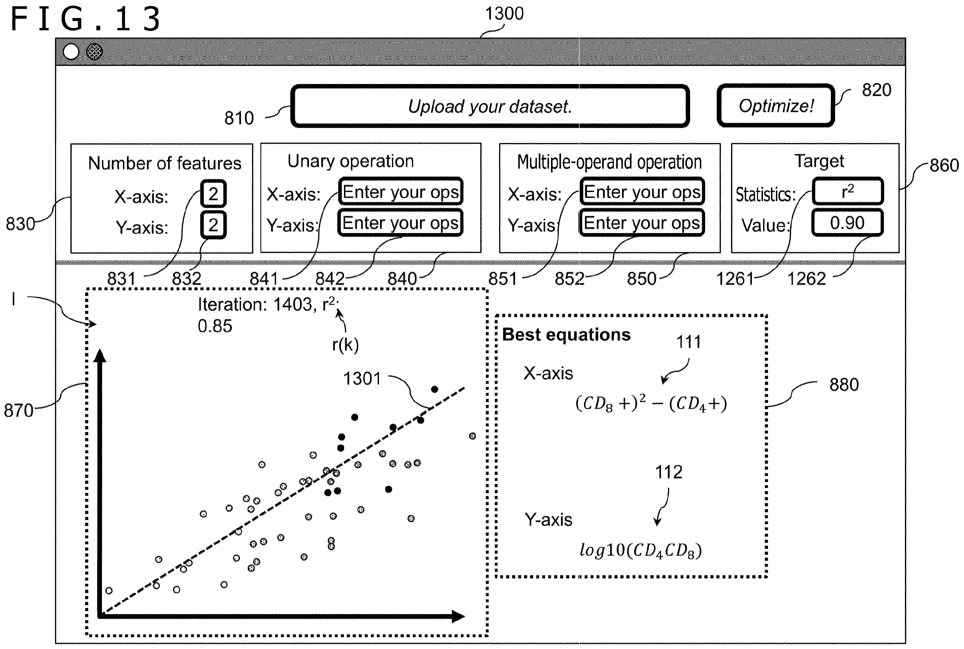

FIG. 13 is an explanatory diagram depicting an example of an input/output screen displayed on the output device 204 of the data processing apparatus 100 according to the second embodiment. Since the objective variable 1202 is the quantitative variable, a determination coefficient ($r^2$) or a mean square error can be selected as statistics r in a statistic input area 1261. Furthermore, a target precision (for example, "0.90" in FIG. 13) can be input to a target value input area 1262 as a target value of the statistics input to the statistic input area 1261.

Moreover, the image generator 530 adapts a luminance value of each pixel that is the patient data about each patient plotted onto the coordinate space 110 to the magnitude of the objective variable 1202 and determines a shade of the pixel by referring to the objective variables 1202 on the data memory 500. In a case in which the value of the objective variable 1202 is great, the pixel indicating the patient data concerned is rendered in a bright color.

On the other hand, in a case in which the value of the objective variable 1202 is small, the pixel indicating the patient data concerned is rendered in a dark color. The image generator 530 stores the generated image data I(t) in the data memory 500 and outputs the image data I(t) to the controller 550. Furthermore, the image generator 530 generates a regression line 1301 by referring to the patient data of the image data I(t). In this way, according to the second embodiment, the data processing apparatus 100 is also applicable to regression analysis.

Furthermore, the example of using the number of immune cells of each patient as the object-to-be analyzed data has been described in the first and second embodiments. However, the object-to-be-analyzed data is not limited to such biological information and is also applicable to, for example, stocks. For example, the object to be analyzed may be issues of companies, the patient ID 301 may be an issue ID, and the factor group 303 may be company information containing a net profit, the number of employees, a sales volume, and the like of each company. Moreover, in a case of the first embodiment, the objective variable 302 may indicate a rise or a fall of the issue concerned or whether it is possible to buy the issue. Furthermore, in a case of the second embodiment, the objective variable (quantitative variable) 1202 may be a stock price of the issue concerned.

Furthermore, the data processing apparatuses 100 according to the first and second embodiments can be configured as described in (1) to (13) below.

(1) For example, the data processing apparatus 100 includes: a storage section, the X-axis modulation unit 510, the Y-axis modulation unit 520, and the image generator 530. The data memory 500, which is an example of the storage section, stores an object-to-be-analyzed data group (object-to-be-analyzed DB 104) having the factor group 303 and the objective variable 302 per object to be analyzed. The X-axis modulation unit 510 modulates a first factor (x1, x2) and outputs a first modulation result (X coordinate value of each patient data) per object to be analyzed. The Y-axis modulation unit 520 modulates a second factor (y1, y2) and outputs a second modulation result (Y coordinate value of each patient data) per object to be analyzed. The image generator 530 assigns a coordinate point (each patient data) representing the first modulation result from the X-axis modulation unit 510 and the second modulation result from the Y-axis modulation unit 520 to the coordinate space 110 per object to be analyzed, the coordinate space 110 being specified by the X-axis corresponding to the first factor and the Y-axis corresponding to the second factor, and generates the image data I(t) obtained by assigning information (for example, pixel color) associated with the objective variable 302 of the object to be analyzed corresponding to the coordinate point to the coordinate point.
The user can thereby easily perform discrimination and regression analysis of the patient data groups according to a combination of a plurality of factors by referring to the image data I(t).
(2) Furthermore, in (1) described above, the storage section stores the pattern table 208 containing types of elements out of at least either the types of factors or the types of the modulation methods for the factors, and the data processing apparatus 100 further includes the controller 550. The controller 550 generates the control signal a(t) for causing the X-axis modulation unit 510 to select a first element and the Y-axis modulation unit 520 to select a second element using the pattern table 208, and controls the X-axis modulation unit 510 and the Y-axis modulation unit 520 on the basis of the control signal a(t).

The controller 550 can thereby control the X-axis modulation unit 510 and the Y-axis modulation unit 520 in response to the elements stored in the pattern table 208, formulate the equations 111 and 112, and output the coordinate values (patient data) . The image generator 530 can, therefore, generate the image data I(t) by plotting the coordinate values (patient data) onto the coordinate space 110.

(3) Moreover, in (2) described above, the pattern table 208 may contain the types of the factors, and the controller 550 may generate the control signal a (t) for causing the X-axis modulation unit 510 to select the first factor and the Y-axis modulation unit 520 to select the second factor using the pattern table 208, and control the X-axis modulation unit 510 and the Y-axis modulation unit 520 on the basis of the control signal a(t).

The controller 550 can thereby generate the control signal a(t) specifying predetermined modulation methods or modulation methods designated by the user 103 and control the X-axis modulation unit 510 and the Y-axis modulation unit 520 on the basis of the control signal a (t) even in a case in which the pattern table 208 stores the types of the factors such as CD4+, CD8+, ..., CTLA-4, and MIP-1$\beta$ and does not store the types of the modulation methods.

(4) Furthermore, in (2) described above, the pattern table 208 may contain the types of the modulation methods, and the controller 550 may generate the control signal a(t) for causing the X-axis modulation unit 510 to select a first modulation method and the Y-axis modulation unit 520 to select a second modulation method using the pattern table 208, and control the X-axis modulation unit 510 and the Y-axis modulation unit 520 on the basis of the control signal a (t) .

The controller 550 can thereby generate the control signal a(t) specifying predetermined factors or factors designated by the user 103 and control the X-axis modulation unit 510 and the Y-axis modulation unit 520 on the basis of the control signal a(t) even in a case in which the pattern table 208 stores the modulation methods such as the non-modulation, the sign change, the logarithmic transformation, the absolute value transformation, the exponentiation, and the four arithmetic operations and does not store the types of the factors.

(5) Moreover, in (2) described above, the pattern table 208 may contain the types of the factors and the types of the modulation methods for the factors, and the controller 550 may generate the control signal a(t) for causing the X-axis modulation unit 510 to select one element out of at least either the first factor or the first modulation method, and causing the Y-axis modulation unit 520 to select one element out of at least either the second factor or the second modulation method using the pattern table 208, and control the X-axis modulation unit 510 and the Y-axis modulation unit 520 on the basis of the control signal a(t).

The controller 550 can thereby comprehensively generate the control signal a(t) having a combination of the factors and the modulation methods, and contribute to increasing generation patterns of the image data I(t).

(6) Furthermore, in (2) described above, the controller 550 may update part of elements in the control signal a(t) by referring to the pattern table 208, and control the X-axis modulation unit 510 and the Y-axis modulation unit 520 by the updated control signal a(t), and the image generator 530 may generate the image data I(t+1) by the controller 550 controlling the X-axis modulation unit 510 and the Y-axis modulation unit 520 based on the updated control signal a (t) .

The image generator 530 can thereby generate the image data I(t+1) reflective of the action of the value based on the updated control signal a(t), and the controller 550 can thereby take the next action in such a state of the image data I(t+1).

(7) Moreover, in (6) described above, the controller 550 may include the Q* network 601 that outputs the one-dimensional array z(t) indicating the value of each element in the pattern table 208 in a case of taking a first action in a first state on the basis of the learning parameter $\theta*$ when the image data I(t+1) is assumed as the first state and a first element group contained in the control signal a(t) is assumed as the first action, update an element (for example, "CD8+" of the control ID: 514) in the control signal a(t), the element corresponding to a specific value (for example, 0.9) in the one-dimensional array z(t) indicating the value of each element in the pattern table 208, to a specific element (for example, "MIP-1$\beta$" of the element number 100) corresponding to the specific value (for example, 0.9) in the pattern table 208, and control the X-axis modulation unit 510 and the Y-axis modulation unit 520 on the basis of the updated control signal a(t).

The image generator 530 can thereby generate the image data I(t+1) reflective of the action of the specific value based on the updated control signal a(t), and the controller 550 can thereby take the next action in such a state of the image data I(t+1).

(8) Furthermore, in (7) described above, the specific value may be a value indicating a maximum value in the one-dimensional array z(t) indicating the value of each element in the pattern table 208.

The image generator 530 can thereby generate the image data I (t+1) reflective of the action of the maximum value based on the updated control signal a(t), and the controller 550 can thereby take the next action in such a state of the image data I(t+1). Therefore, it is possible for the image generator 530 to generate the image data I(t) maximizing the action, and possible to facilitate the discrimination and the regression analysis of the patient data groups according to a combination of a plurality of factors, and to realize automation and speed enhancing of data processing.

(9) Moreover, in (7) described above, the data processing apparatus 100 includes the evaluator 540 that evaluates the objective variable 302 on the basis of the first modulation result (X coordinate value of each patient data), the second modulation result (Y coordinate value of each patient data), and information (for example, pixel color) associated with the objective variable 302. The controller 550 includes the Q network 602 that outputs the one-dimensional array z (t) indicating the value of each element in the pattern table 208 in a case of taking a second action in a second state on the basis of the learning parameter $\theta$ when input image data is assumed as the second state and a second element group contained in the updated control signal a(t) is assumed as the second action. The controller 550 may calculate a value of the first action as the supervisory data y(j) by adding, as a reward, statistics r(j) that is an evaluation result by the evaluator 540 to an output result in a case of inputting the image data I(t+1) to the Q network 602, update the learning parameter $\theta$ on the basis of the supervisory data y(j) and an output result in a case of inputting the image data I(t) to the Q network 602, and update the learning parameter $\theta^*$ to the updated learning parameter $\theta$.

It is thereby possible to achieve optimization of the $Q^*$ network 601, and identify the higher value element from the one-dimensional array z(t) output by the $Q^*$ network 601. Therefore, it is possible to facilitate the discrimination and the regression analysis of the patient data groups according to a combination of a plurality of factors, and to realize automation and speed enhancing of data processing.

(10) Furthermore, in (1) described above, the data processing apparatus 100 includes: the evaluator 540; and an output section (output device 204 or communication IF 205) . The evaluator 540 may evaluate the objective variable 302 on the basis of the first modulation result (X coordinate value of each patient data), the second modulation result (Y coordinate value of each patient data), and the information (for example, pixel color) associated with the objective variable 302. The output section may output image data I(j) in a displayable fashion in a case in which the statistics r(j) that is the evaluation result by the evaluator 540 is, for example, equal to or greater than the target value input to the target value input area 862.

The data processing apparatus 100 can thereby narrow down image data to the image data I(j) necessary for the user 103.

(11) Moreover, in (10) described above, the objective variable 302 may be information for classifying the object-to-be-analyzed data group, the image generator 530 may generate the discrimination demarcation line 113 for discriminating the coordinate points by the objective variable 302, and the output section may output the discrimination demarcation line 113 to the image data I(j) in a displayable fashion. The user can thereby visually identify a demarcation for discriminating a coordinate point group corresponding to each objective variable 302.

(12) Furthermore, in (11) described above, the factor group 303 may be biological information and the objective variable 302 may information indicating the medicinal effect. The user can thereby easily stratify patients into the patient data group (response group) on which the medicine takes effect and the patient data group (non-response group) on which the medicine does not take effect by the discrimination demarcation line 113.

(13) Moreover, in (11) described above, the objective variable 302 may be the quantitative variable, the image generator 530 may generate the regression line 1301 on the basis of the coordinate points and the objective variable 302, and the output section may output the regression line 1301 to the image data I(j) in a displayable fashion. The data processing apparatus 100 can be thereby applied to regression analysis.

The present invention is not limited to the embodiments described above and encompasses various modifications and equivalent configurations within the meaning of the accompanying claims. For example, the above-mentioned embodiments have been described in detail for describing the present invention so that the present invention is easy to understand, and the present invention is not always limited to the embodiments having all the described configurations. Furthermore, a part of configurations of one embodiment may be replaced by configurations of the other embodiment. Moreover, the configurations of the other embodiment may be added to the configurations of the one embodiment. Further, for part of the configurations of each embodiment, addition, deletion, or replacement may be made of the other configurations.

Moreover, a part of or all of the configurations, the functions, the processing sections, processing means, and the like described above may be realized by hardware by being designed, for example, as an integrated circuit, or may be realized by software by causing a processor to interpret and execute programs that realize the functions.

Information in programs, tables, files, and the like for realizing the functions can be stored in a storage device such as a memory, a hard disk, or a solid state drive (SSD), or in a recording medium such as an integrated circuit (IC) card, a secure digital (SD) card, or a digital versatile disc (DVD) .

Furthermore, control lines or information lines considered to be necessary for the description are illustrated and all the control lines or the information lines necessary for implementation are not always illustrated. In actuality, it may be contemplated that almost all the configurations are mutually connected.

Features described in this specification and/or in the claims and/or shown in a figure shall be deemed combinable with and amongst each other also if their combination is not expressly described, to the extent that the combination is technically feasible. Features described in a certain context, embodiment, figure or claim shall be deemed separable from this claim, context, embodiment or figure and shall be deemed combinable with every other figure, claim, context or embodiment, to the extent that it is technically feasible. Embodiments and figures shall not be understood as being meant necessarily exclusive against each other. Descriptions of a method or procedure or a method step or a procedural step shall be understood also as description of means and/or possibly as a data carrier holding program instructions of executable code adapted for implementing the method or procedure or method step or procedural step, and vice versa.

**Claims**

1. A data processing apparatus comprising:

   a storage section that stores an object-to-be-analyzed data group having factors and an objective variable per object to be analyzed;
   a first modulation section that modulates a first factor and outputs a first modulation result per object to be analyzed;
   a second modulation section that modulates a second factor and outputs a second modulation result per object to be analyzed; and
   a generation section that assigns a coordinate point representing the first modulation result from the first modulation section and the second modulation result from the second modulation section to a coordinate space per object to be analyzed, the coordinate space being specified by a first axis corresponding to the first factor and a second axis corresponding to the second factor, and that generates first image data obtained by assigning information associated with the objective variable of the object to be analyzed corresponding to the coordinate point to the coordinate point.

2. The data processing apparatus according to claim 1,
   the storage section storing pattern information containing types of element out of at least either types of factors or types of modulation methods for the factors, the data processing apparatus further comprising:
   a control section that generates a control signal for causing the first modulation section to select a first element and the second modulation section to select a second element using the pattern information, and that controls the first modulation section and the second modulation section on the basis of the control signal.

3. The data processing apparatus according to claim 2, wherein
   the pattern information contains the types of the factors, and
   the control section generates a control signal for causing the first modulation section to select the first factor and the second modulation section to select the second factor using the pattern information, and that controls the first modulation section and the second modulation section on a basis of the control signal.

4. The data processing apparatus according to claim 2, wherein
   the pattern information contains the types of the modulation methods, and
   the control section generates a control signal for causing the first modulation section to select a first modulation method and the second modulation section to select a second modulation method using the pattern information, and that controls the first modulation section and the second modulation section on a basis of the control signal.

5. The data processing apparatus according to claim 2, wherein
   the pattern information contains the types of the factors and the types of the modulation methods for the factors,
   the control section generates a control signal for causing the first modulation section to select one element out of at least either the first factor or a first modulation method, and causing the second modulation section to select one element out of at least either the second factor or a second modulation method, and that controls the first modulation section and the second modulation section on a basis of the control signal.

6. The data processing apparatus according to claim 2, wherein
   the control section updates part of elements in the control signal by referring to the pattern information, and controls the first modulation section and the second modulation section by an updated control signal in which the part of elements has been updated, and
   the generation section generates second image data by the control section controlling the first modulation section and the second modulation section based on the updated control signal.

7. The data processing apparatus according to claim 6, wherein

the control section includes a first action value function that outputs a value of each element in the pattern information in a case of taking a first action in a first state on a basis of a first learning parameter when the first image data is assumed as the first state and a first element group contained in the control signal is assumed as the first action, updates an element in the control signal, the element corresponding to a specific value output from the first action value function among values of elements in the pattern information, to a specific element corresponding to the specific value in the pattern information, and controls the first modulation section and the second modulation section on a basis of the updated control signal.

8.  The data processing apparatus according to claim 7, wherein
the specific value is a value indicating a maximum value among the value of each element in the pattern information.

9.  The data processing apparatus according to claim 7, further comprising:

an evaluation section that evaluates the objective variable on a basis of the first modulation result, the second modulation result, and information associated with the objective variable, wherein
the control section includes a second action value function that outputs the value of each element in the pattern information in a case of taking a second action in a second state on a basis of a second learning parameter when input image data is assumed as the second state and a second element group contained in the updated control signal is assumed as the second action, calculates a value of the first action as supervisory data by adding, as a reward, an evaluation result by the evaluation section to an output result in a case of inputting the second image data to the second action value function, updates the second learning parameter on a basis of the supervisory data and an output result in a case of inputting the first image data to the second action value function, and updates the first learning parameter based on an updated second learning parameter.

10. The data processing apparatus according to claim 1, further comprising:

an evaluation section that evaluates the objective variable on a basis of the first modulation result, the second modulation result, and information associated with the objective variable; and
an output section that outputs the first image data in a displayable fashion in a case in which an evaluation result by the evaluation section is equal to or greater than a target value.

11. The data processing apparatus according to claim 10, wherein
the objective variable is information for classifying the object-to-be-analyzed data group,
the generation section generates a discrimination demarcation line for discriminating the coordinate point by the objective variable, and
the output section outputs the discrimination demarcation line to the first image data in a displayable fashion.

12. The data processing apparatus according to claim 11, wherein
the factors are biological information, and the objective variable is information indicating a medicinal effect.

13. The data processing apparatus according to claim 11, wherein
the objective variable is a quantitative variable,
the generation section generates a regression line based on the coordinate point and the objective variable, and
the output section outputs the regression line to the first image data in a displayable fashion.

14. A data processing method executed by a data processing apparatus accessible to a storage section storing an object-to-be-analyzed data group having factors and an objective variable per object to be analyzed, the data processing method comprising:

first modulation processing for modulating a first factor and outputting a first modulation result per object to be analyzed;
second modulation processing for modulating a second factor and outputting a second modulation result per object to be analyzed; and
generation processing for assigning a coordinate point representing the first modulation result by the first modulation processing and the second modulation result by the second modulation processing to a coordinate space per object to be analyzed, the coordinate space being specified by a first axis corresponding to the first factor and a second axis corresponding to the second factor, and generating image data obtained by assigning information associated with the objective variable of the object to be analyzed corresponding to the coordinate point to the coordinate point.

15. A data processing program for a processor accessible to a storage section storing an object-to-be-analyzed data group having factors and an objective variable per object to be analyzed, the data processing program comprising:

first modulation processing for modulating a first factor and outputting a first modulation result per object to be analyzed;
second modulation processing for modulating a second factor and outputting a second modulation result per object to be analyzed; and
generation processing for assigning a coordinate point representing the first modulation result by the first modulation processing and the second modulation result by the second modulation processing to a coordinate space per object to be analyzed, the coordinate space being specified by a first axis corresponding to the first factor and a second axis corresponding to the second factor, and generating image data obtained by assigning information associated with the objective variable of the object to be analyzed corresponding to the coordinate point to the coordinate point.

# FIG.1

| PATIENT ID | MEDICINAL EFFECT | 100 TYPES OF IMMUNE CELLS |
|---|---|---|
| ... | ... | ... |

104 OBJECT-TO-BE-ANALYZED DB

103

READ

105 ELEMENT GROUP

(1)

EQUATION FORMULATION AI (6)

101

REWARD: PREDICTION PRECISION

(4)

DISCRIMINATOR

102

100

(3)

COORDINATE VALUES

112

113

110

$Yopy_0\{Yopy_1(y_1)\ BopY\ Yopy_2(y_2)\}$

$Xopx_0\{Xopx_1(x_1)\ BopX\ Xopx_2(x_2)\}$

111

(2) PLOT (●, ■) VALUES CALCULATED BY SUBSTITUTING NUMBER OF CELLS INTO X-AXIS EQUATION AND VALUES CALCULATED BY SUBSTITUTING NUMBER OF CELLS INTO Y-AXIS EQUATIONS ONTO COORDINATE SPACE

(5) IMAGE DATA ABOUT COORDINATE SPACE

I

LEGEND
● TRUE PATIENT GROUP (RESPONSE GROUP) ON WHICH MEDICINE TAKES EFFECT
■ TRUE PATIENT GROUP (NON-RESPONSE GROUP) ON WHICH MEDICINE DOES NOT TAKE EFFECT

EP 3 792 931 A1

FIG.2

FIG.3

| PATIENT ID | OBJECTIVE VARIABLE (RESPONSE/NON-RESPONSE) | CD4+ | CD8+ | ... | CTLA-4 | MIP-1β |
|---|---|---|---|---|---|---|
| | | | | | Log10, 1/2 | Log10 |
| 1 | 1 | 372 | 303 | ... | 89 | 78 |
| ... | ... | ... | ... | ... | ... | ... |
| 49 | 0 | 128 | 390 | ... | 173 | 33 |
| 50 | 1 | 12 | 180 | ... | 201 | 190 |

104
OBJECT-TO-BE-ANALYZED DB

301　302　303　304

# F I G . 4

| CONTROL ID | 1 | 2 | 3 | 4 | 5 | 6 | 7 | ... | 99 | 100 |
|---|---|---|---|---|---|---|---|---|---|---|
| 513 | CD4+ | CD8+ | ... | ... | ... | ... | ... | ... | CTLA-4 | MIP-1β |
| 514 | CD4+ | CD8+ | ... | ... | ... | ... | ... | ... | CTLA-4 | MIP-1β |
| 515 | NON-MODULATION | SIGN CHANGE | ABSOLUTE VALUE | LOGARITHM | 1/2 | 2 | 3 | | | |
| 516 | NON-MODULATION | SIGN CHANGE | ABSOLUTE VALUE | LOGARITHM | 1/2 | 2 | 3 | | | |
| 517 | + | - | X | / | | | | | | |
| 518 | NON-MODULATION | SIGN CHANGE | ABSOLUTE VALUE | LOGARITHM | 1/2 | 2 | 3 | | | |
| 523 | CD4+ | CD8+ | ... | ... | ... | ... | ... | ... | CTLA-4 | MIP-1β |
| 524 | CD4+ | CD8+ | ... | ... | ... | ... | ... | ... | CTLA-4 | MIP-1β |
| 525 | NON-MODULATION | SIGN CHANGE | ABSOLUTE VALUE | LOGARITHM | 1/2 | 2 | 3 | | | |
| 526 | NON-MODULATION | SIGN CHANGE | ABSOLUTE VALUE | LOGARITHM | 1/2 | 2 | 3 | | | |
| 527 | + | - | X | / | | | | | | |
| 528 | NON-MODULATION | SIGN CHANGE | ABSOLUTE VALUE | LOGARITHM | 1/2 | 2 | 3 | | | |

401

402

208

PATTERN TABLE

EP 3 792 931 A1

# F I G . 5

IMAGE PROCESSING CIRCUIT

X-AXIS MODULATION UNIT 518

- MODULATOR
- MULTIOPERATOR 517
- 510
- 511
- 512
- 516
- MODULATOR 515
- MODULATOR
- MULTIPLEXER 513
- MULTIPLEXER 514

208

PATTERN TABLE

530

IMAGE GENERATOR

500 — DATA MEMORY

540 — EVALUATOR

550 — CONTROLLER

207

Y-AXIS MODULATION UNIT

- 528 — MODULATOR
- MULTIOPERATOR 527
- 520
- 521
- 522
- 526
- MODULATOR 525
- MODULATOR
- MULTIPLEXER 523
- MULTIPLEXER 524

EP 3 792 931 A1

# FIG.6

CONTROLLER 550

NETWORK UNIT 600

601 Q* NETWORK
LEARNING PARAMETER θ*

602 Q NETWORK
LEARNING PARAMETER θ

603 RANDOM UNIT

630 LEARNING PARAMETER UPDATE UNIT

631 GRADIENT CALCULATION UNIT

620 REPLAY MEMORY

DATA PACK

STATISTICS : r(t)

IMAGE DATA : I(t), I(t+1)

CONTROL SIGNAL : a(t)

STOP SIGNAL : K(t)

D(t)

EP 3 792 931 A1

# FIG.7

$a(t)$

| | x1 | x2 | opx1 | opx2 | opxa | opxb | y1 | y2 | opy1 | opy2 | opya | opyb |
|---|---|---|---|---|---|---|---|---|---|---|---|---|
| CONTROL ID | 513 | 514 | 515 | 516 | 517 | 518 | 523 | 524 | 525 | 526 | 527 | 528 |
| ACTION | CD4+ | CD8+ | NON-MODULATION | NON-MODULATION | / | log10 | CD4+ | | 1/2 | NON-MODULATION | + | NON-MODULATION |

401
701

X-AXIS $\quad \log_{10}\left(\dfrac{CD_4+}{CD_8+}\right)$

111

Y-AXIS $\quad \sqrt{CD_4+}$

112

# FIG.8

800

Upload your dataset.    810    Optimize!    820

860

**Number of features** — 830
X-axis: 2
Y-axis: 2
831  832

**Unary operation**
X-axis: Enter your ops
Y-axis: Enter your ops
841  842  840

**Multiple-operand operation**
X-axis: Enter your ops
Y-axis: Enter your ops
851  852  850

**Target**
Statistics : AUC
Value: 0.8
861  862

870

Iteration: 240, AUC: 0.85
r(k)
113

**Best equations**    111

X-axis
$$\log 10(\frac{CD_4 +}{CD_8 +})$$

880

112
Y-axis
$$\sqrt{CD_4 +}$$

EP 3 792 931 A1

# F I G . 9

```
START
   │
   ▼
S901 ─┐ EXECUTE PROCESSING BY X-
      │ AXIS DATA LOAD MODULES
   │
   ▼
S902 ─┐ EXECUTE PROCESSING BY
      │ X-AXIS MULTIOPERATOR
   │
   ▼
S903 ─┐ EXECUTE PROCESSING
      │ BY X-AXIS MODULATOR
   │
   ▼
S1003, S1006 ─┐  S904 ─┐ EXECUTE PROCESSING BY Y-
             │         │ AXIS DATA LOAD MODULES
   │
   ▼
S905 ─┐ EXECUTE PROCESSING BY
      │ Y-AXIS MULTIOPERATOR
   │
   ▼
S906 ─┐ EXECUTE PROCESSING
      │ BY Y-AXIS MODULATOR
   │
   ▼
S907 ─┐ EXECUTE PROCESSING
      │ BY IMAGE GENERATOR
   │
   ▼
  END
```

# FIG. 10

S1000 ~ START

```
S1001 ─ EXECUTE INITIALIZATION

S1002 ─ INITIALIZE CONTROLLER

S1003 ─ EXECUTE IMAGE DATA I(t)
         GENERATION PROCESSING

S1004 ─ UPDATE CONTROL SIGNAL a(t)

S1005 ─ EXECUTE PROCESSING
         BY EVALUATOR

S1006 ─ EXECUTE IMAGE DATA I(t+1)
         GENERATION PROCESSING

S1007 ─ STORE DATA PACK D(t)
         IN REPLAY MEMORY
```

```
S1008 ─ CONTINUE IMAGE GENERATION ──Yes── S1004

         │ No

S1009 ─ UPDATE SUPERVISED SIGNAL y(j)      UPDATE TIME
                                           STEP t AS IN t=t+1
S1010 ─ EXECUTE LEARNING CALCULATION

S1011 ─ CONTINUE ANALYSIS ──Yes── S1002

         │ No

S1012 ─ STORE DATA PACK CONTAINING STATISTICS r(k) EQUAL TO
         OR GREATER THAN TARGET VALUE IN STORAGE DEVICE

S1013 ─ DISPLAY RESULT

S1014 ─ END
```

# FIG.11

EP 3 792 931 A1

| | CONTROL ID: 513 | | | | CONTROL ID: 514 | | | | CONTROL ID: 528 | | | | | | | |
|---|---|---|---|---|---|---|---|---|---|---|---|---|---|---|---|---|

| ELEMENT NUMBER | 1 | 2 | ... | 99 | 100 | 1 | ... | 100 | ... | 1 | 2 | 3 | 4 | 5 | 6 | 7 |
|---|---|---|---|---|---|---|---|---|---|---|---|---|---|---|---|---|
| ARRAY NUMBER | 1 | 2 | ... | 99 | 100 | 101 | ... | 200 | ... | 444 | 445 | 446 | 447 | 448 | 449 | 450 |
| $z(t)$ | 0.3 | 0.1 | ... | 0.7 | 0.4 | 0.1 | ... | 0.9 | ... | 0.3 | 0.0 | 0.2 | 0.1 | 0.0 | 0.0 | 0.3 |

CD4+ CD8+ ... CTLA-4 MIP-1β CD4+ ... MIP-1β ... NON-MODULATION SIGN CHANGE ABSOLUTE VALUE LOGARITHM 1/2 2 3

EXPONENTS

# F I G . 1 2

EP 3 792 931 A1

| PATIENT ID | OBJECTIVE VARIABLE (QUANTITATIVE VARIABLE) | CD4+ | CD8+ | ... | CTLA-4 | MIP-1β |
|---|---|---|---|---|---|---|
| | | | | | Log10, sqrt | Log10 |
| 1 | 100.0 | 372 | 303 | ... | 89 | 78 |
| ... | ... | ... | ... | ... | ... | ... |
| 49 | 30.4 | 128 | 390 | ... | 173 | 33 |
| 50 | 55.3 | 12 | 180 | ... | 201 | 190 |

OBJECT-TO-BE-ANALYZED DB

1200

301

1202

303

304

# FIG.13

**810** Upload your dataset.   **820** Optimize!

**830**

| Number of features | Unary operation | Multiple-operand operation | Target |
|---|---|---|---|
| X-axis: 2 | X-axis: Enter your ops | X-axis: Enter your ops | Statistics: $r^2$ |
| Y-axis: 2 | Y-axis: Enter your ops | Y-axis: Enter your ops | Value: 0.90 |

**831  832   841  842  840   851  852  850   1261  1262**

**860**

Iteration: 1403, $r^2$: 0.85

r(k)

**1301**

**870**

**Best equations**

X-axis  **111**

$$(CD_8 +)^2 - (CD_4 +)$$

**880**

Y-axis  **112**

$$log10(CD_4 CD_8)$$

EP 3 792 931 A1

Europäisches
Patentamt

European
Patent Office

Office européen
des brevets

# EUROPEAN SEARCH REPORT

**Application Number**

EP 20 19 3762

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/017174 A1 (ATREYA RAJA [DE] ET AL) 16 January 2014 (2014-01-16) * figures 2D, 3A, 3B, 3C, 3D * * page 3, paragraph 0021 - paragraph 0022 * * page 22, paragraph 0200 - page 24, paragraph 0215 * | 1-15 | INV. G16H50/20 G16H50/70 ADD. G16H40/20 |
| X | WO 97/44752 A1 (MEDICAL SCIENCE SYS INC [US]) 27 November 1997 (1997-11-27) * page 1, line 11 - line 16 * * page 5, line 14 - page 6, line 11 * * page 19, line 22 - page 22, line 7; figures 12, 13 * | 1-15 | |
| X | KR 102 018 006 B1 (LEE JONG KYUN [KR]) 4 September 2019 (2019-09-04) * page 6, paragraph 0001 - paragraph 0003 * * page 12, paragraph 0055 - page 17, paragraph 0086; figures 1-6 * | 1-15 | |

**TECHNICAL FIELDS SEARCHED (IPC)**

G16H

The present search report has been drawn up for all claims

| Place of search | Date of completion of the search | Examiner |
|---|---|---|
| Munich | 26 January 2021 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 19 3762

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2021

| Patent document cited in search report | | Publication date | Patent family member(s) | | Publication date |
|---|---|---|---|---|---|
| US 2014017174 | A1 | 16-01-2014 | AU | 2012346861 A1 | 19-06-2014 |
| | | | CA | 2857597 A1 | 06-06-2013 |
| | | | EP | 2786156 A2 | 08-10-2014 |
| | | | HK | 1202628 A1 | 02-10-2015 |
| | | | US | 2014017174 A1 | 16-01-2014 |
| | | | WO | 2013080050 A2 | 06-06-2013 |
| WO 9744752 | A1 | 27-11-1997 | AU | 3140897 A | 09-12-1997 |
| | | | WO | 9744752 A1 | 27-11-1997 |
| KR 102018006 | B1 | 04-09-2019 | CN | 110023760 A | 16-07-2019 |
| | | | KR | 20190088403 A | 26-07-2019 |
| | | | WO | 2018221820 A1 | 06-12-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019164352 A

**Non-patent literature cited in the description**

- **SUBRAHMANYAM, PRIYANKA B. et al.** Distinct predictive biomarker candidates for response to anti-CTLA-4 and anti-PD-1 immunotherapy in melanoma patients. *Journal for immunotherapy of cancer 6.1,* 2018, 18